# EUROPEAN PATENT APPLICATION

(11) **EP 3 779 455 A1**
(43) Date of publication of application: **17.02.2021**
(21) Application number: 19382710.2
(22) Date of filing: 14.08.2019
(51) Int. Cl.: G01N 33/92

(54) **LIPID SIGNATURES FOR DETERMINING THE OUTCOME OF EMBRYO IMPLANTATION DURING IN VITRO FERTILIZATION**

(71) Applicant: Asociación Centro de Investigación Cooperativa en Biociencias - CIC bioGUNE, 48160 Derio (ES); REPRODALIA, S.L., 48013 Bilbao (ES)
(72) Inventor: MATORRAS WEINIG, Roberto, E-48013 Bilbao, Bizkaia (ES); ELORTZA BASTERRIKA, Félix, E-48160 Derio - Vizcaya (ES); MARTÍNEZ ARRANZ, Ibon, E-48160 Derio, Bizkaia (ES)
(74) Representative: ABG Intellectual Property Law, S.L.

(57) **Abstract**

The present invention relates to new methods for predicting the outcome of in vitro fertilization (IVF) procedures, as well as selecting the subjects to undergo IVF treatments. It also relates to methods for selecting IVF treatments and decision-making in said treatments as well as wash solutions for use in the treatment of infertility.

## Description

### FIELD OF THE INVENTION

The invention relates to a non-invasive method for the determination of endometrial receptivity to embryo implantation. The method is especially applicable for determining status fertility of a mammalian female, preferably, a woman.

### BACKGROUND OF THE INVENTION

More than 12 % of the reproductive population worldwide has difficulties in conceiving and a majority of the women affected of infertility are between the ages of 15 and 44. Infertility is the most common medical disorder among women in these ages. The couples might be helped by an assisted reproductive technique, such as in vitro fertilization (IVF). IVF is a process by which egg cells are fertilized by sperm in vitro outside the womb. IVF is a major treatment in infertility when other methods of assisted reproductive technology have failed. The process typically involves hormonally controlling the ovulatory process, removing ova from the woman's ovaries and letting sperm fertilize them in a fluid or culture medium to form zygotes that are matured into embryos. A formed embryo/s is/are then transferred to the patient's uterus with the intent to establish a successful pregnancy.

Implantation of the transferred embryo/s involves a synchronized crosstalk between a receptive endometrium and a functional blastocyst. This phenomenon can only take place during the window of implantation, a self-limited period of endometrial receptivity spanning between days 19 and 23 of the menstrual cycle (women). In normal menstrual cycles this is achieved through the local effects of ovarian estrogens and progesterone, which induce a series of cellular and molecular events in the endometrium leading to appropriate endometrial receptivity.

The pregnancy rate, i.e. the success rate for pregnancy following IVF, has increased a lot the last few years clinical studies report an average pregnancy rate of up to 70 % in industrial countries, possible after several trials. In this sense, a lot of research has been invested in the field of trying to increase this pregnancy rate even further.

The era of molecular and cellular biology, and the development of new analytical techniques, has found more consistent predictors of a receptive endometrium. In this regard, although genetic and proteomic analysis of sample biopsies have added new exciting tools for the assessment of endometrial receptivity, these approaches, however, have not yet rendered results applicable to the everyday-IVF clinical practice, since no single specific factor has been identified to be crucial for implantation in humans. Indeed a biopsy-based test cannot be performed in the same cycle where the embryo transfer is performed, because of the risk of impairing implantation. Thus, biopsy-based tests always need that the embryo be cryopreserved and the transfer be performed in a delayed cycle.

The analysis of endometrial secretions is a new, non-disruptive possibility for the investigation of endometrial receptivity. Importantly, the aspiration of endometrial fluid does not affect pregnancy rates, thus opening the field for a non-invasive application. Although some biochemical markers have been disclosed by assessing endometrial receptivity, there is a need in the art for specific and reliable molecular biomarkers for the detection of endometrial receptivity to embryo implantation in women, which can be used in the clinic.

### SUMMARY OF THE INVENTION

The authors of the invention have surprisingly found that specific lipid molecules are significantly different in endometrial fluid aspirate in implantative versus non-implantative IVF cycles. Thus, the specific lipidomic profile in the endometrial fluid aspirate could be used as a tool in the planning of embryo transfer strategies. In this context, in the presence of an adverse lipidomic profile, embryo transfer could be canceled (cryopreserving the embryos) or delayed, or perhaps the endometrial fluid could be flushed to wash up the adverse lipids, or even one could take the decision of increasing the number of embryos to be transferred. On the other hand, if the lipidomic profile is favorable, a single embryo transfer could be performed. The specific lipidomic profile, alone or combined with other markers could be the basis for developing a quick system that should allow clinicians to make IVF treatment decisions in less than 24 hours.

Thus, in a first aspect the invention relates to a method for predicting the outcome of, or assessing the chances of success of, in vitro fertilisation (IVF) in a subject, wherein the method comprises determining, in an endometrial fluid sample from the subject, the expression level of at least one lipid marker selected from the group consisting of
- Lysoglycerophosphoethanolamine (20:5);
- 1-ether, 2-acylglycerophosphoethanolamine (16:1e/20:5);
- Diacylglycerophosphocholine (40:8);
- Diacylglycerophosphocholine (18:0/18:1);
- Diacylglycerophosphocholine (18:0/18:2);
- Lysoglycerophosphoethanolamine (22:5/0:0);
- Docosapentaenoic acid (22:5n-6); and/ or
- Lysoglycerophosphoethanolamine (18:2/0:0),
   wherein
- a decrease in the expression level of Diacylglycerophosphocholine (40:8) and Docosapentaenoic acid (22:5n-6) with respect to a reference value, and
- an increase in the expression level of Lysoglycerophosphoethanolamine (20:5); 1-ether, 2-acylglycerophosphoethanolamine (16:1e/20:5); Diacylglycerophosphocholine (18:0/18:1); Diacylglycerophosphocholine (18:0/18:2); Lysoglycerophosphoethanolamine (22:5/0:0); and Lysoglycerophosphoethanolamine (18:2/0:0) with respect to a reference value
is indicative of an increased chance of a positive outcome of IVF for the subject.

In a second aspect, the invention relates to a method for selecting a subject for implantation with at least one embryo during IVF, wherein the method comprises determining, in an endometrial fluid sample from the subject, the expression level of at least one lipid marker selected from the group consisting of
- Lysoglycerophosphoethanolamine (20:5);
- 1-ether, 2-acylglycerophosphoethanolamine (16:1e/20:5);
- Diacylglycerophosphocholine (40:8);
- Diacylglycerophosphocholine (18:0/18:1);
- Diacylglycerophosphocholine (18:0/18:2);
- Lysoglycerophosphoethanolamine (22:5/0:0);
- Docosapentaenoic acid (22:5n-6); and/ or
- Lysoglycerophosphoethanolamine (18:2/0:0),
   wherein
- a decrease in the expression level of Diacylglycerophosphocholine (40:8) and Docosapentaenoic acid (22:5n-6) with respect to a reference value, and
- an increase in the expression level of Lysoglycerophosphoethanolamine (20:5); 1-ether, 2-acylglycerophosphoethanolamine (16:1e/20:5); Diacylglycerophosphocholine (18:0/18:1); Diacylglycerophosphocholine (18:0/18:2); Lysoglycerophosphoethanolamine (22:5/0:0); and Lysoglycerophosphoethanolamine (18:2/0:0) with respect to a reference value
   is indicative that the subject is selected for implantation with one embryo,
   and wherein
- an increase in the expression level of Diacylglycerophosphocholine (40:8) and Docosapentaenoic acid (22:5n-6) with respect to a reference value, and
- a decrease in the expression level of Lysoglycerophosphoethanolamine (20:5); 1-ether, 2-acylglycerophosphoethanolamine (16:1e/20:5); Diacylglycerophosphocholine (18:0/18:1); Diacylglycerophosphocholine (18:0/18:2); Lysoglycerophosphoethanolamine (22:5/0:0); and Lysoglycerophosphoethanolamine (18:2/0:0) with respect to a reference value
is indicative that the subject is selected for implantation with two or more embryos, or that the embryos are frozen and IVF is postponed.

In a third aspect method for selecting an IVF treatment for a subject, wherein the method comprises determining, in an endometrial fluid sample from the subject, the expression level of at least one lipid marker selected from the group consisting of
- Lysoglycerophosphoethanolamine (20:5);
- 1-ether, 2-acylglycerophosphoethanolamine (16:1e/20:5);
- Diacylglycerophosphocholine (40:8);
- Diacylglycerophosphocholine (18:0/18:1);
- Diacylglycerophosphocholine (18:0/18:2);
- Lysoglycerophosphoethanolamine (22:5/0:0);
- Docosapentaenoic acid (22:5n-6); and/ or
- Lysoglycerophosphoethanolamine (18:2/0:0),
   wherein
- a decrease in the expression level of Diacylglycerophosphocholine (40:8) and Docosapentaenoic acid (22:5n-6) with respect to a reference value, and
- an increase in the expression level of Lysoglycerophosphoethanolamine (20:5); 1-ether, 2-acylglycerophosphoethanolamine (16:1e/20:5); Diacylglycerophosphocholine (18:0/18:1); Diacylglycerophosphocholine (18:0/18:2); Lysoglycerophosphoethanolamine (22:5/0:0); and Lysoglycerophosphoethanolamine (18:2/0:0) with respect to a reference value is indicative that an IVF treatment comprising the implantation of one embryo is selected for the subject,
   and wherein
- an increase in the expression level of Diacylglycerophosphocholine (40:8) and Docosapentaenoic acid (22:5n-6) with respect to a reference value, and
- a decrease in the expression level of Lysoglycerophosphoethanolamine (20:5); 1-ether, 2-acylglycerophosphoethanolamine (16:1e/20:5); Diacylglycerophosphocholine (18:0/18:1); Diacylglycerophosphocholine (18:0/18:2); Lysoglycerophosphoethanolamine (22:5/0:0); and Lysoglycerophosphoethanolamine (18:2/0:0) with respect to a reference value
is indicative that an IVF treatment comprising the implantation of two or more embryos is selected for the subject, or that the embryos are frozen and IVF is postponed.

In a fourth aspect the invention relates to the use of reagents specific for the determination of the expression levels of
- Lysoglycerophosphoethanolamine (20:5);
- 1-ether, 2-acylglycerophosphoethanolamine (16:1e/20:5);
- Diacylglycerophosphocholine (40:8);
- Diacylglycerophosphocholine (18:0/18:1);
- Diacylglycerophosphocholine (18:0/18:2);
- Lysoglycerophosphoethanolamine (22:5/0:0);
- Docosapentaenoic acid (22:5n-6); and/ or
- Lysoglycerophosphoethanolamine (18:2/0:0),
for predicting the outcome of, or assessing the chances of success of, IVF in a subject.

In a fifth aspect the invention relates to an endometrial fluid wash solution for use in the treatment of infertility in a subject, wherein the wash solution is suitable for
- decreasing the expression level of Diacylglycerophosphocholine (40:8) and Docosapentaenoic acid (22:5n-6) with respect to a reference value, and
- increasing the expression level of Lysoglycerophosphoethanolamine (20:5); 1-ether, 2-acylglycerophosphoethanolamine (16:1e/20:5); Diacylglycerophosphocholine (18:0/18:1); Diacylglycerophosphocholine (18:0/18:2); Lysoglycerophosphoethanolamine (22:5/0:0); and Lysoglycerophosphoethanolamine (18:2/0:0) with respect to a reference value.

In a sixth aspect, the invention relates to an endometrial fluid wash solution comprising an effective amount of
- Lysoglycerophosphoethanolamine (20:5);
- 1-ether, 2-acylglycerophosphoethanolamine (16:1e/20:5);
- Diacylglycerophosphocholine (40:8);
- Diacylglycerophosphocholine (18:0/18:1);
- Diacylglycerophosphocholine (18:0/18:2);
- Lysoglycerophosphoethanolamine (22:5/0:0);
- Docosapentaenoic acid (22:5n-6); and/ or
- Lysoglycerophosphoethanolamine (18:2/0:0).

### DESCRIPTION OF THE FIGURES

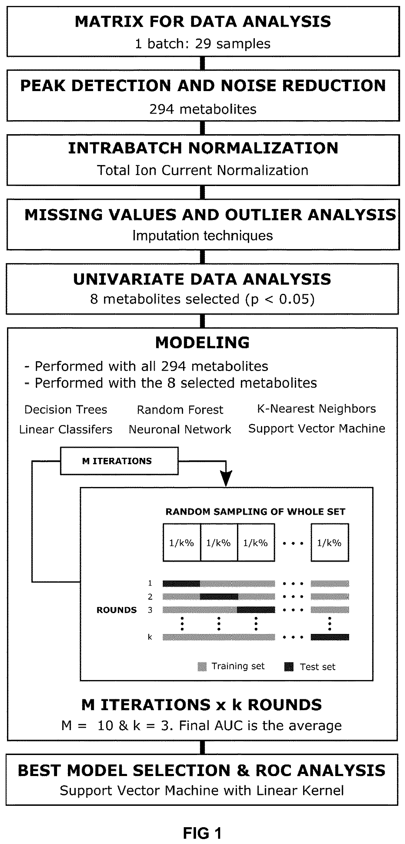

**Figure 1****. Data analysis workflow.** Detailed flowchart of data analysis procedure followed. Data matrix includes 29 samples and 294 metabolites (lipid species). Peak intensity for each metabolite was normalized to the sum of the peak intensities within each sample. Once normalized, outlier analysis was performed and imputation techniques were used for the missing values. Univariate analysis (Student's t-test) was then carried out. Eight metabolites were found to be significantly altered (p<0.05) in the non-implantative endometrium group, comparing to the implantative endometrium. Different modeling approaches were performed taking into account all metabolites and only the metabolites selected after univariate analysis: Decision Trees, Random Forest, K-Nearest Neighbors, Linear Classifiers, Neuronal Network and Support Vector Machine. A three-fold Cross Validation was performed with a 10 resampling for each modeling. After ROC analysis, Support Vector Machine model with linear kernel was selected.

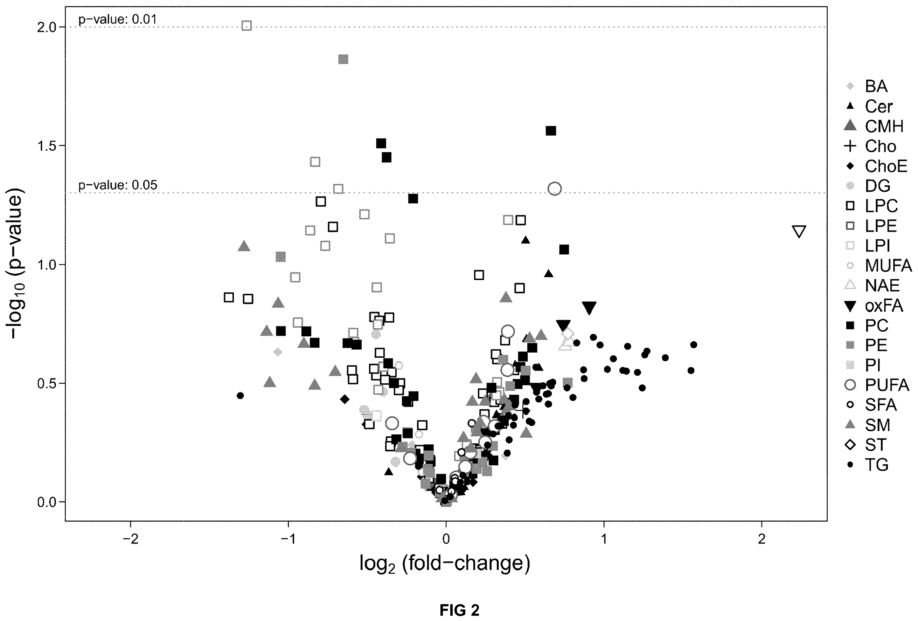

**Figure 2****. Differential metabolite levels in implantative compared** to **non-implantative EFA.** Volcano plot representation indicating the -log₁₀ (p-value) *vs.* log₂ (fold-change) of each individual metabolic ion features for the comparison implantative *vs.* non-implantative endometrium. BA, bile acid; Cer, ceramide; CMH, monohexosylceramide; Cho, cholesterol; ChoE, cholesteryl ester; DG, diglyceride; LPC, lysophosphatidylcholine; LPE, lysophosphatidylethanolamine; LPI, lysophosphatidylinositol; MUFA, monounsaturated fatty acid; NAE, N-acyl ethanolamine; oxFA, oxidized fatty acid; PC, phosphatidylcholine; PE, phosphatidylethanolamine; PI, phosphatidylinositol; PUFA, polyunsaturated fatty acid; SFA, saturated fatty acid; SM, sphingomyelin; ST, steroid; TG, triglyceride.

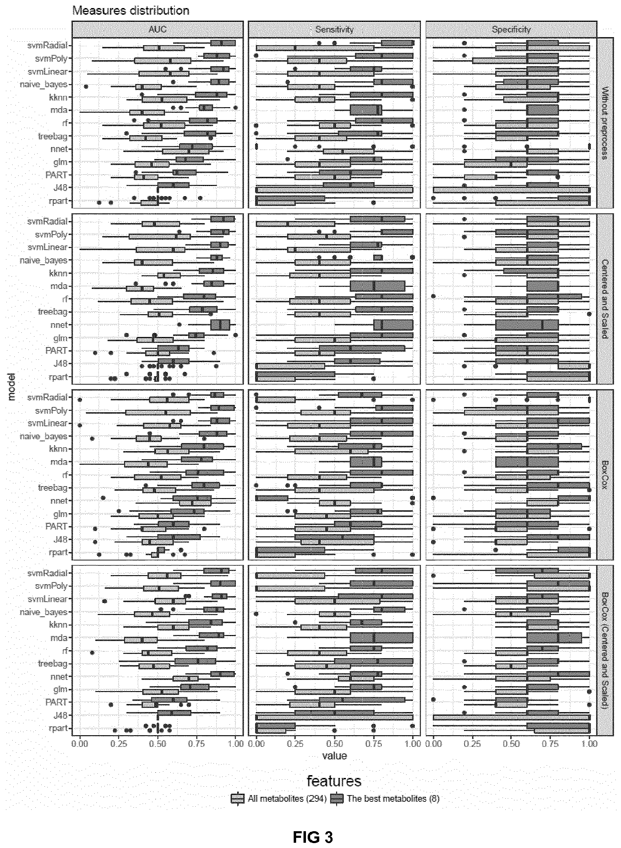

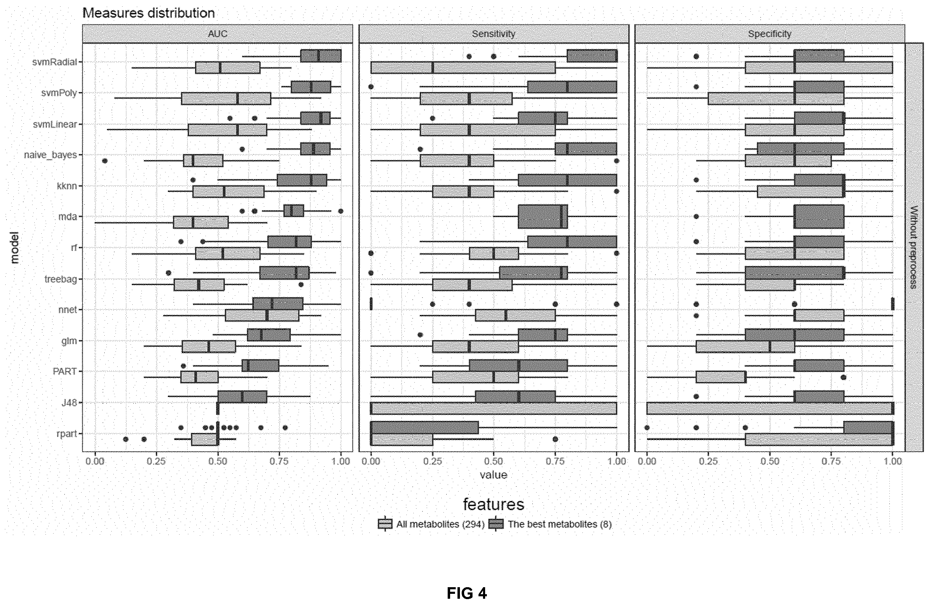

**Figure 3****. Summary of all performed models for the prediction of the endometrial implantative status with different pre-processing approaches:** without pre-processing, centered and scaled, Box-Cox, and Box-Cox and centered and scaled. Boxplots show the distribution of Area Under the ROC Curve, sensitivity and specificity in the Cross-Validation process for the following methodologies: Decision Trees (treebag, rpart, PART, and J48), Linear Classifiers (Naïve-Bayes -naive_bayes-, Generalized Linear Models -glm- and Flexible Discriminant Analysis -mda-), K-Nearest Neighbors -kknn-, Neuronal Network -nnet-, Random Forest -rf- and Support Vector Machine with several kernels: Linear -svmLinear-, Radial -svmradial- and Polynomial - svmPoly-. All these methodologies were tested including all detected variables (a total of 294 lipids) or the eight significant metabolites selected under the t-test criterion (p < 0.05). Four different pre-processing of the data were also applied: without pre-processing (upper panel), centered and scaled, Box-Cox, and Box-Cox and centered and scaled (lower panel). Models are ordered from the best to worst results in AUC (used to select the optimal model using the largest value) with no pre-processing (according to Figure 4).
**Figure 4****. Summary of all performed models for the prediction of the endometrial implantative status.** Boxplots show the distribution of AUC, sensitivity and specificity in the Cross-Validation process for the following methodologies: Decision Trees (treebag, rpart, PART, and J48), Linear Classifiers (Naïve-Bayes -naïve_bayes-, Generalized Linear Models -glm- and Flexible Discriminant Analysis -mda-), K-Nearest Neighbors -kknn-, Neuronal Network -nnet-, Random Forest -rf- and Support Vector Machine with several kernels: Linear -svmLinear-, Radial -svmradial- and Polynomial - svmPoly-. No preprocessing was performed in these models. All these methodologies were tested including all detected variables (a total of 294 lipids) or the eight significant metabolites selected under the t-test criterion (p < 0.05). Models are ordered from the best to worst results in AUC.

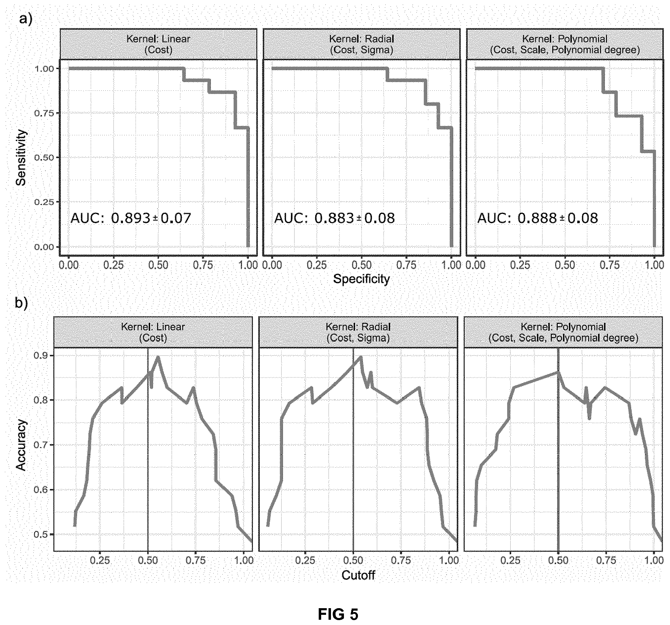

**Figure 5****. ROC Analysis of the Support Vector Machine models.** A) ROC Curve by kernel. Analysis performed for the optimal tuning parameters: Linear kernel: C = 0.28; Radial kernel: C = 2.74 and sigma = 0.017; Polynomial kernel: C = 1.09, scale = 0.024 and degree = 1. B) Accuracy by kernel. A cut-off at 0.5 is considered

### DETAILED DESCRIPTION OF THE INVENTION

As explained above, the authors of the invention have surprisingly found that specific lipid molecules are significantly different in endometrial fluid aspirate in implantative versus non-implantative IVF cycles. Thus, the specific lipidomic profile in the endometrial fluid aspirate could be used as a tool in the planning of embryo transfer strategies. In this context, in the presence of an adverse lipidomic profile, embryo transfer could be canceled (cryopreserving the embryos) or delayed, or perhaps the endometrial fluid could be flushed to wash up the adverse lipids, or even one could take the decision of increasing the number of embryos to be transferred. On the other hand, if the lipidomic profile is favorable, a single embryo transfer could be performed. The specific lipidomic profile, alone or combined with other markers could be the basis for developing a quick system that should allow clinicians to make IVF treatment decisions in less than 24 hours.

Thus, in a first aspect the invention relates to a method for predicting the outcome of, or assessing the chances of success of, in vitro fertilisation (IVF) in a subject, wherein the method comprises determining, in an endometrial fluid sample from the subject, the expression level of at least one lipid marker selected from the group consisting of
- Lysoglycerophosphoethanolamine (20:5);
- 1-ether, 2-acylglycerophosphoethanolamine (16:1e/20:5);
- Diacylglycerophosphocholine (40:8);
- Diacylglycerophosphocholine (18:0/18:1);
- Diacylglycerophosphocholine (18:0/18:2);
- Lysoglycerophosphoethanolamine (22:5/0:0);
- Docosapentaenoic acid (22:5n-6); and/ or
- Lysoglycerophosphoethanolamine (18:2/0:0),
   wherein
- a decrease in the expression level of Diacylglycerophosphocholine (40:8) and Docosapentaenoic acid (22:5n-6) with respect to a reference value, and
- an increase in the expression level of Lysoglycerophosphoethanolamine (20:5); 1-ether, 2-acylglycerophosphoethanolamine (16:1e/20:5); Diacylglycerophosphocholine (18:0/18:1); Diacylglycerophosphocholine (18:0/18:2); Lysoglycerophosphoethanolamine (22:5/0:0); and Lysoglycerophosphoethanolamine (18:2/0:0) with respect to a reference value
   is indicative of an increased chance of a positive outcome of IVF for the subject.

As used herein, the expression "predicting the outcome" refers to a method for determining the probability of a female subject being in a period receptive for embryo implantation (i.e., a period when embryo implantation is highly likely). The skilled person in the art will understand that said prediction cannot be correct for the 100% of the female subjects under study. However, said expression requires that the prediction method provides correct results for a statistically significant portion of female subjects. This can be determined by using standard statistical techniques such as the confidence intervals determination, p value determination, t test of Student, or the Mann- Whitney test, as explained by Dowdy and Wearden, Statistics for Research, John Wiley & Sons, New York 1983. Suitable confidence intervals are, at least, 50%, at least 60%, at least 70%, at least 80%, preferably, at least 90%, or most preferable, at least 95%. Preferably, p values are 0.2, 0.1, or, most preferably 0.05.

The term "implantation" as used herein refers to the stage of pregnancy at which the embryo adheres to the wall of the uterus. The expression "period receptive for embryo implantation" or "implantation window" encompasses the window of time during which the uterine endometrium is receptive to the conceptus; in humans, this occurs in the secretory stage of the menstrual cycle. Implantation is defined as days 6-8 post the day of the luteinising hormone (LH) surge. The implantation window can be estimated on the basis of a regular menstrual pattern as approximately 7 days before the expected first day of the menstrual period, i.e., it corresponds, therefore, to days 19-21 of an ideal menstrual cycle of 28 days in humans. Similar cycles have been disclosed in other mammals, so that the method of the invention could be adapted to any mammalian female.

The term "in vitro fertilization" is used herein to refer to an assisted reproductive technology (ART) commonly referred to as IVF. IVF is the process of fertilization by extracting eggs, retrieving a sperm sample, and then combining an egg and sperm outside of the body (i.e., in a laboratory dish, or similar). The embryo(s) is then transferred to the uterus.

The term "subject", as used herein, refers to a mammalian female. The term "mammalian" includes any mammal, for example, humans and non-human primates, cattle, goat, sheep, horses, pigs, dogs, etc. In a particular embodiment, said mammalian female is a human female or non-human primate. In a preferred embodiment said mammalian female is a woman (i.e., a human female).

As used herein, the term "endometrial fluid" refers to a secretion of the glandular epithelium which contains all the compounds secreted to the lumen of the uterus. Briefly, the endometrium constitutes the surface tissue which lines the uterine wall (myometrium or middle layer of the uterine wall consisting of smooth muscle cells and supporting stromal and vascular tissue) of a mammalian female or, in other words, the inner membrane of the mammalian uterus. The endometrium is extremely sensitive to the hormones estrogen and progesterone and is composed of several functional layers. The tissue architecture of the endometrium comprises an external cell layer, which constitutes the epithelium, which is in direct contact with the lumen of the uterus, and an internal cell layer, denominated stroma, which constitutes around 80% of the endometrium thickness. Further, the endometrium contains blood vessels and specialized cells, which confer its functional characteristics to the endometrium. The endometrial epithelium constitutes a continuous cell layer which is organized in two regions: luminal epithelium (epithelial cells that line the surface of the endometrium) and glandular epithelium (epithelial cells that form glands beneath the surface of the endometrium), said regions being physically different and molecularly recognizable. The luminal epithelium represents a physical barrier against the pathogens attacks and develops structures, denominated pinopodes, in its apical surface, which are responsible of the absorption of uterine lumen material; however, the glandular epithelium is responsible for the secretions, which form the endometrial fluid, which contains all compounds secreted to the lumen of the uterus.

The term "implantative cycle" corresponds to a cycle were implantation takes place. The term "non-implantative cycle" corresponds to a cycle where, in spite of transferring one/ two embryos, implantation does not occur. In all implantative cycles, the endometrium is implantative. However, in a non-implantative cycle the endometrium may be non-implantative or the cause could be a bad quality embryo (or both).

The method of the present invention comprises determining, in an endometrial fluid sample from the subject, the expression level of at least one lipid marker selected from the group consisting of
- Lysoglycerophosphoethanolamine (20:5);
- 1-ether, 2-acylglycerophosphoethanolamine (16:1e/20:5);
- Diacylglycerophosphocholine (40:8);
- Diacylglycerophosphocholine (18:0/18:1);
- Diacylglycerophosphocholine (18:0/18:2);
- Lysoglycerophosphoethanolamine (22:5/0:0);
- Docosapentaenoic acid (22:5n-6); and/ or
- Lysoglycerophosphoethanolamine (18:2/0:0),

As used herein, the term "expression level" refers to the value of a parameter that measures the extent of the concentration of the particular lipid marker in the endometrial fluid sample (i.e., an endometrial fluid aspirate) from the subject. The term can refer to a quantitative amount (e.g., weight or moles), a semi-quantitative amount, a relative amount (e.g., weight % or mole % within class or a ratio), a concentration, and the like.

The term "Lysoglycerophosphoethanolamine (20:5)", also known as "LPE(20:5)", refers to a lysoglycerophosphoethanolamine with formula C₂₅H₄₂NO₇P, having the common name PE(20:5(5Z,8Z,11Z,14Z,17Z)/0:0) with Lipid Maps accession number LMGP02050027 or the common name PE(0:0/20:5(5Z,8Z,11Z,14Z,17Z)) with Lipid Maps accession number LMGP02050053.

The term "1-ether, 2-acylglycerophosphoethanolamine (16:1e/20:5)", also known as "PE(16:1e/20:5)", refers to a 1-ether, 2-acylglycerophosphoethanolamine with formula C₄₁H₇₂NO₇P, having the common name PE(P-16:0/20:5(5Z,8Z,11Z,14Z,17Z) with Lipid Maps accession number LMGP02030028, or the common name PE(o-16:1/20:5).

The term "Diacylglycerophosphocholine (40:8)", also known as "PC(40:8)", refers to a diacylglycerophosphocholine with formula C₄₈H₈₀NO₈P, having the common name PC(20:4(5E,8E,11E,14E)/20:4(5E,8E,11E,14E)) with Lipid Maps accession number LMGP01011047 or the common name PC(20:4(5Z,8Z,11Z,14Z)/20:4(5Z,8Z,11Z, 14Z)) with Lipid Maps accession number LMGP01011052.

The term "Diacylglycerophosphocholine (18:0/18:1)", also known as PC(18:0/18:1), refers to a diacylglycerophosphocholine with formula C₄₄H₈₆NO₈P, having the common name PC(18:0/18:1(11Z)) with Lipid Maps accession number LMGP01010750; the common name PC(18:0/18:1(12Z)) with Lipid Maps accession number LMGP01010751; the common name PC(18:0/18:1(13Z)) with Lipid Maps accession number LMGP01010753; the common name PC(18:0/18:1(16Z)) with Lipid Maps accession number LMGP01010754; the common name PC(18:0/18:1(6Z)) with Lipid Maps accession number LMGP01010756; the common name PC(18:0/18:1(7Z)) with Lipid Maps accession number LMGP01010758; the common name PC(18:0/18:1(9E)) with Lipid Maps accession number LMGP01010759; or the common name PC(18:0/18:1(9Z)) with Lipid Maps accession number LMGP01010761.

The term "Diacylglycerophosphocholine (18:0/18:2)", also known as "PC(18:0/18:2)", refers to a diacylglycerophosphocholine with formula C₄₄H₈₄NO₈P, having the common name PC(18:0/18:2(10Z,12Z)) with Lipid Maps accession number LMGP01010764; having the common name PC(18:0/18:2(2E,4E)) with Lipid Maps accession number LMGP01010765; having the common name PC(18:0/18:2(6Z,9Z)) with Lipid Maps accession number LMGP01010766; or having the common name PC(18:0/18:2(9Z,12Z)) with Lipid Maps accession number LMGP01010768.

The term "Lysoglycerophosphoethanolamine (22:5/0:0)", also known as "LPE(22:5/0:0)", refers to a lysoglycerophosphoethanolamine with formula C₂₇H₄₆NO₇P, having the common name PE(22:5(4Z,7Z,10Z,13Z,16Z)/0:0) with Lipid Maps accession number LMGP02050069; or having the common name PE(22:5(7Z,10Z,13Z,16Z,19Z)/0:0) with Lipid Maps accession number LMGP02050070.

The term "Docosapentaenoic acid (22:5n-6)", also known as "DPA(22:5N-6)", refers to a omega 6 polyunsaturated fatty acid with formula C₂₂H₃₄O₂ having the common name 4,7,10,13,16-docosapentaenoic acid with Lipid Maps accession number LMFA01030182.

The term "Lysoglycerophosphoethanolamine (18:2/0:0)", also known as "LPE(18:2/0:0)", refers to a lysoglycerophosphoethanolamine with formula C₂₃H₄₄NO₇P having the common name PE(18:2(9Z,12Z)/0:0) with Lipid Maps accession number LMGP02050011.

The method of the invention includes the determination of at least one the lipid markers referred above, which includes de determination of one, two, three, four, five, six, seven, or eight of the lipid biomarkers of the invention, in any possible combination.

Thus, in a particular embodiment of the invention, the method comprises determining, in an endometrial fluid sample from the subject, the expression level of one lipid marker selected from:
- Lysoglycerophosphoethanolamine (20:5);
- 1-ether, 2-acylglycerophosphoethanolamine (16:1e/20:5);
- Diacylglycerophosphocholine (40:8);
- Diacylglycerophosphocholine (18:0/18:1);
- Diacylglycerophosphocholine (18:0/18:2);
- Lysoglycerophosphoethanolamine (22:5/0:0);
- Docosapentaenoic acid (22:5n-6); and
- Lysoglycerophosphoethanolamine (18:2/0:0),

In another particular embodiment of the invention, the method comprises determining, in an endometrial fluid sample from the subject, the expression level of two lipid markers selected from:
- LPE(20:5) and DPA(22:5n-6);
- LPE(20:5) and PE(16:1e/20:5);
- LPE(20:5) and PC(40:8);
- LPE(20:5) and PC(18:0/18:2);
- LPE(20:5) and LPE(22:5/0:0);
- LPE(20:5) and LPE(18:2/0:0);
- LPE(20:5) and PC(18:0/18:1);
- PE(16:1e/20:5) and PC(40:8);
- PE(16:1e/20:5) and PC(18:0/18:2);
- PE(16:1e/20:5) and DPA(22:5n-6);
- PE(16:1e/20:5) and LPE(18:2/0:0);
- PE(16:1e/20:5) and LPE(22:5/0:0);
- PE(16:1e/20:5) and PC(18:0/18:1);
- PC(40:8) and PC(18:0/18:2);
- PC(40:8) and LPE(22:5/0:0);
- PC(40:8) and LPE(18:2/0:0);
- PC(40:8) and DPA(22:5n-6);
- PC(40:8) and PC(18:0/18:1);
- PC(18:0/18:1) and PC(18:0/18:2);
- PC(18:0/18:1) and DPA(22:5n-6);
- PC(18:0/18:1) and LPE(18:2/0:0);
- PC(18:0/18:1) and LPE(22:5/0:0);
- PC(18:0/18:2) and LPE(22:5/0:0);
- PC(18:0/18:2) and DPA(22:5n-6);
- PC(18:0/18:2) and LPE(18:2/0:0);
- LPE(22:5/0:0) and DPA(22:5n-6);
- LPE(22:5/0:0) and LPE(18:2/0:0); and
- DPA(22:5n-6) and LPE(18:2/0:0).

In yet another particular embodiment of the invention, the method comprises determining, in an endometrial fluid sample from the subject, the expression level of three lipid markers selected from:
- LPE(20:5), PE(16:1e/20:5), DPA(22:5n-6);
- LPE(20:5), PC(40:8), DPA(22:5n-6);
- PE(16:1e/20:5), PC(18:0/18:2), DPA(22:5n-6);
- LPE(20:5), PC(40:8), PC(18:0/18:2);
- PC(40:8), PC(18:0/18:2), DPA(22:5n-6);
- PC(18:0/18:2), LPE(22:5/0:0), DPA(22:5n-6);
- PC(40:8), PC(18:0/18:2), LPE(18:2/0:0);
- PE(16:1e/20:5), PC(40:8), PC(18:0/18:2);
- PE(16:1e/20:5), PC(18:0/18:2), LPE(22:5/0:0);
- PC(18:0/18:2), LPE(22:5/0:0), LPE(18:2/0:0);
- PC(40:8), PC(18:0/18:2), LPE(22:5/0:0);
- PE(16:1e/20:5), PC(18:0/18:1), PC(18:0/18:2);
- LPE(20:5), PE(16:1e/20:5), PC(18:0/18:2);
- LPE(20:5), PC(18:0/18:2), LPE(18:2/0:0);
- PC(18:0/18:2), DPA(22:5n-6), LPE(18:2/0:0);
- LPE(20:5), PC(18:0/18:2), DPA(22:5n-6);
- PC(18:0/18:1), PC(18:0/18:2), DPA(22:5n-6);
- LPE(20:5), PC(18:0/18:2), LPE(22:5/0:0);
- PC(18:0/18:1), PC(18:0/18:2), LPE(22:5/0:0);
- PE(16:1e/20:5), PC(18:0/18:2), LPE(18:2/0:0);
- LPE(20:5), PC(18:0/18:1), PC(18:0/18:2);
- PC(40:8), LPE(22:5/0:0), LPE(18:2/0:0);
- LPE(22:5/0:0), DPA(22:5n-6), LPE(18:2/0:0);
- PC(40:8), PC(18:0/18:1), PC(18:0/18:2);
- LPE(20:5), LPE(22:5/0:0), LPE(18:2/0:0);
- LPE(20:5), PE(16:1e/20:5), PC(40:8);
- PC(18:0/18:1), PC(18:0/18:2), LPE(18:2/0:0);
- PE(16:1e/20:5), LPE(22:5/0:0), LPE(18:2/0:0);
- LPE(20:5), PC(18:0/18:1), LPE(18:2/0:0);
- PC(18:0/18:1), LPE(22:5/0:0), LPE(18:2/0:0);
- LPE(20:5), PC(18:0/18:1), DPA(22:5n-6);
- LPE(20:5), PC(40:8), PC(18:0/18:1);
- PC(40:8), PC(18:0/18:1), DPA(22:5n-6);
- PC(18:0/18:1), DPA(22:5n-6), LPE(18:2/0:0);
- PE(16:1e/20:5), PC(18:0/18:1), DPA(22:5n-6);
- PE(16:1e/20:5), PC(40:8), PC(18:0/18:1);
- LPE(20:5), PC(40:8), LPE(18:2/0:0);
- PC(40:8), PC(18:0/18:1), LPE(18:2/0:0);
- LPE(20:5), PC(18:0/18:1), LPE(22:5/0:0);
- LPE(20:5), PE(16:1e/20:5), LPE(18:2/0:0);
- PE(16:1e/20:5), PC(18:0/18:1), LPE(22:5/0:0);
- LPE(20:5), PE(16:1e/20:5), PC(18:0/18:1);
- PC(40:8), PC(18:0/18:1), LPE(22:5/0:0);
- PC(18:0/18:1), LPE(22:5/0:0), DPA(22:5n-6);
- PE(16:1e/20:5), PC(18:0/18:1), LPE(18:2/0:0);
- LPE(20:5), DPA(22:5n-6), LPE(18:2/0:0);
- LPE(20:5), LPE(22:5/0:0), DPA(22:5n-6);
- PE(16:1e/20:5), PC(40:8), DPA(22:5n-6);
- PC(40:8), DPA(22:5e-6), LPE(18:2/0:0);
- PC(40:8), LPE(22:5/0:0), DPA(22:5n-6);
- PE(16:1e/20:5), LPE(22:5/0:0), DPA(22:5n-6);
- PE(16:1e/20:5), DPA(22:5n-6), LPE(18:2/0:0);
- PE(16:1e/20:5), PC(40:8), LPE(18:2/0:0);
- PE(16:1e/20:5), PC(40:8), LPE(22:5/0:0);
- LPE(20:5), PC(40:8), LPE(22:5/0:0); and
- LPE(20:5), PE(16:1E/20:5), LPE(22:5/0:0).

In a further particular embodiment of the invention, the method comprises determining, in an endometrial fluid sample from the subject, the expression level of four lipid markers selected from:
- LPE(20:5), PE(16:1e/20:5), PC(40:8), DPA(22:5n-6);
- LPE(20:5), PE(16:1e/20:5), PC(18:0/18:2), DPA(22:5n-6);
- LPE(20:5), PC(40:8), PC(18:0/18:2), DPA(22:5n-6);
- LPE(20:5), PC(40:8), PC(18:0/18:2), LPE(18:2/0:0);
- LPE(20:5), PC(18:0/18:2), LPE(22:5/0:0), LPE(18:2/0:0);
- PC(40:8), PC(18:0/18:2), LPE(22:5/0:0), DPA(22:5n-6);
- PC(18:0/18:2), LPE(22:5/0:0), DPA(22:5n-6), LPE(18:2/0:0);
- PE(16:1e/20:5), PC(18:0/18:2), LPE(22:5/0:0), LPE(18:2/0:0);
- PC(40:8), PC(18:0/18:1), PC(18:0/18:2), LPE(18:2/0:0);
- LPE(20:5), PC(18:0/18:1), PC(18:0/18:2), LPE(18:2/0:0);
- LPE(20:5), PE(16:1e/20:5), PC(40:8), PC(18:0/18:2);
- LPE(20:5), PE(16:1e/20:5), PC(18:0/18:2), LPE(22:5/0:0);
- PE(16:1e/20:5), PC(18:0/18:1), PC(18:0/18:2), DPA(22:5n-6);
- LPE(20:5), PC(18:0/18:2), LPE(22:5/0:0), DPA(22:5n-6);
- PC(40:8), PC(18:0/18:2), LPE(22:5/0:0), LPE(18:2/0:0);
- PE(16:1e/20:5), PC(40:8), PC(18:0/18:2), LPE(18:2/0:0);
- PE(16:1e/20:5), PC(18:0/18:1), PC(18:0/18:2), LPE(22:5/0:0);
- LPE(20:5), PC(40:8), PC(18:0/18:2), LPE(22:5/0:0);
- PE(16:1e/20:5), PC(18:0/18:2), LPE(22:5/0:0), DPA(22:5n-6);
- LPE(20:5), PE(16:1e/20:5), PC(18:0/18:2), LPE(18:2/0:0);
- PC(40:8), PC(18:0/18:1), PC(18:0/18:2), DPA(22:5n-6);
- PE(16:1e/20:5), PC(40:8), PC(18:0/18:2), DPA(22:5n-6);
- LPE(20:5), PC(18:0/18:2), DPA(22:5n-6), LPE(18:2/0:0);
- PE(16:1e/20:5), PC(40:8), PC(18:0/18:2), LPE(22:5/0:0);
- PC(18:0/18:1), PC(18:0/18:2), LPE(22:5/0:0), DPA(22:5n-6);
- LPE(20:5), PC(18:0/18:1), PC(18:0/18:2), DPA(22:5n-6);
- LPE(20:5), PE(16:1e/20:5), LPE(22:5/0:0), LPE(18:2/0:0);
- PE(16:1e/20:5), PC(18:0/18:2), DPA(22:5n-6), LPE(18:2/0:0);
- LPE(20:5), PC(40:8), PC(18:0/18:1), PC(18:0/18:2);
- PE(16:1e/20:5), PC(40:8), LPE(22:5/0:0), LPE(18:2/0:0);
- PC(40:8), PC(18:0/18:2), DPA(22:5n-6), LPE(18:2/0:0);
- PC(40:8), PC(18:0/18:1), PC(18:0/18:2), LPE(22:5/0:0);
- PE(16:1e/20:5), PC(40:8), PC(18:0/18:1), PC(18:0/18:2);
- PC(40:8), LPE(22:5/0:0), DPA(22:5n-6), LPE(18:2/0:0);
- LPE(20:5), PC(18:0/18:1), PC(18:0/18:2), LPE(22:5/0:0);
- PC(18:0/18:1), PC(18:0/18:2), DPA(22:5n-6), LPE(18:2/0:0);
- LPE(20:5), PC(40:8), LPE(22:5/0:0), LPE(18:2/0:0);
- LPE(20:5), LPE(22:5/0:0), DPA(22:5n-6), LPE(18:2/0:0);
- PE(16:1e/20:5), PC(18:0/18:1), PC(18:0/18:2), LPE(18:2/0:0);
- LPE(20:5), PE(16:1e/20:5), PC(18:0/18:1), PC(18:0/18:2);
- PE(16:1e/20:5), PC(40:8), PC(18:0/18:1), LPE(18:2/0:0);
- PC(18:0/18:1), PC(18:0/18:2), LPE(22:5/0:0), LPE(18:2/0:0);
- LPE(20:5), PE(16:1e/20:5), PC(18:0/18:1), LPE(18:2/0:0);
- PC(40:8), PC(18:0/18:1), DPA(22:5n-6), LPE(18:2/0:0);
- LPE(20:5), PC(40:8), PC(18:0/18:1), LPE(18:2/0:0);
- PE(16:1e/20:5), LPE(22:5/0:0), DPA(22:5n-6), LPE(18:2/0:0);
- PE(16:1e/20:5), PC(18:0/18:1), LPE(22:5/0:0), LPE(18:2/0:0);
- LPE(20:5), PC(18:0/18:1), DPA(22:5n-6), LPE(18:2/0:0);
- PC(40:8), PC(18:0/18:1), LPE(22:5/0:0), LPE(18:2/0:0);
- PE(16:1e/20:5), PC(40:8), PC(18:0/18:1), LPE(22:5/0:0);
- PC(18:0/18:1), LPE(22:5/0:0), DPA(22:5n-6), LPE(18:2/0:0);
- LPE(20:5), PE(16:1e/20:5), PC(40:8), PC(18:0/18:1);
- LPE(20:5), PC(40:8), PC(18:0/18:1), LPE(22:5/0:0);
- PE(16:1e/20:5), PC(40:8), PC(18:0/18:1), DPA(22:5n-6);
- PE(16:1e/20:5), PC(18:0/18:1), DPA(22:5n-6), LPE(18:2/0:0);
- LPE(20:5), PE(16:1e/20:5), PC(18:0/18:1), DPA(22:5n-6);
- LPE(20:5), PC(18:0/18:1), LPE(22:5/0:0), DPA(22:5n-6);
- LPE(20:5), PC(18:0/18:1), LPE(22:5/0:0), LPE(18:2/0:0);
- PE(16:1e/20:5), PC(18:0/18:1), LPE(22:5/0:0), DPA(22:5n-6);
- LPE(20:5), PC(40:8), PC(18:0/18:1), DPA(22:5n-6);
- PC(40:8), PC(18:0/18:1), LPE(22:5/0:0), DPA(22:5n-6);
- LPE(20:5), PE(16:1e/20:5), PC(18:0/18:1), LPE(22:5/0:0);
- LPE(20:5), PE(16:1e/20:5), PC(40:8), LPE(18:2/0:0);
- LPE(20:5), PC(40:8), DPA(22:5n-6), LPE(18:2/0:0);
- PE(16:1e/20:5), PC(40:8), LPE(22:5/0:0), DPA(22:5n-6);
- LPE(20:5), PE(16:1e/20:5), DPA(22:5n-6), LPE(18:2/0:0);
- LPE(20:5), PE(16:1e/20:5), LPE(22:5/0:0), DPA(22:5n-6);
- LPE(20:5), PC(40:8), LPE(22:5/0:0), DPA(22:5n-6);
- PE(16:1e/20:5), PC(40:8), DPA(22:5n-6), LPE(18:2/0:0); and
- LPE(20:5), PE(16:1e/20:5), PC(40:8), LPE(22:5/0:0).

In still a further particular embodiment of the invention, the method comprises determining, in an endometrial fluid sample from the subject, the expression level of five lipid markers selected from
- PC(40:8), PC(18:0/18:2), LPE(22:5/0:0), DPA(22:5n-6), LPE(18:2/0:0);
- LPE(20:5), PE(16:1e/20:5), PC(18:0/18:1), PC(18:0/18:2), LPE(18:2/0:0);
- LPE(20:5), PC(40:8), PC(18:0/18:2), LPE(22:5/0:0), LPE(18:2/0:0);
- LPE(20:5), PC(40:8), PC(18:0/18:2), LPE(22:5/0:0), DPA(22:5n-6);
- PC(40:8), PC(18:0/18:1), PC(18:0/18:2), DPA(22:5n-6), LPE(18:2/0:0);
- PE(16:1e/20:5), PC(40:8), PC(18:0/18:2), LPE(22:5/0:0), LPE(18:2/0:0);
- PE(16:1e/20:5), PC(18:0/18:2), LPE(22:5/0:0), DPA(22:5n-6), LPE(18:2/0:0);
- PE(16:1e/20:5), PC(40:8), PC(18:0/18:2), DPA(22:5n-6), LPE(18:2/0:0);
- LPE(20:5), PE(16:1e/20:5), PC(40:8), PC(18:0/18:2), DPA(22:5n-6);
- LPE(20:5), PC(40:8), PC(18:0/18:2), DPA(22:5n-6), LPE(18:2/0:0);
- PE(16:1e/20:5), PC(40:8), PC(18:0/18:2), LPE(22:5/0:0), DPA(22:5n-6);
- LPE(20:5), PE(16:1e/20:5), PC(18:0/18:2), LPE(22:5/0:0), LPE(18:2/0:0);
- LPE(20:5), PC(18:0/18:1), PC(18:0/18:2), LPE(22:5/0:0), DPA(22:5n-6);
- PE(16:1e/20:5), PC(40:8), PC(18:0/18:1), PC(18:0/18:2), LPE(18:2/0:0);
- LPE(20:5), PC(18:0/18:2), LPE(22:5/0:0), DPA(22:5n-6), LPE(18:2/0:0);
- PC(18:0/18:1), PC(18:0/18:2), LPE(22:5/0:0), DPA(22:5n-6), LPE(18:2/0:0);
- LPE(20:5), PE(16:1e/20:5), PC(18:0/18:2), LPE(22:5/0:0), DPA(22:5n-6);
- LPE(20:5), PC(18:0/18:1), PC(18:0/18:2), LPE(22:5/0:0), LPE(18:2/0:0);
- LPE(20:5), PE(16:1e/20:5), PC(40:8), PC(18:0/18:1), PC(18:0/18:2);
- LPE(20:5), PC(40:8), PC(18:0/18:1), PC(18:0/18:2), DPA(22:5n-6);
- LPE(20:5), PE(16:1e/20:5), PC(40:8), PC(18:0/18:2), LPE(18:2/0:0);
- PE(16:1e/20:5), PC(40:8), PC(18:0/18:1), PC(18:0/18:2), DPA(22:5n-6);
- PC(40:8), PC(18:0/18:1), PC(18:0/18:2), LPE(22:5/0:0), LPE(18:2/0:0);
- LPE(20:5), PE(16:1e/20:5), PC(40:8), LPE(22:5/0:0), LPE(18:2/0:0);
- LPE(20:5), PE(16:1e/20:5), LPE(22:5/0:0), DPA(22:5n-6), LPE(18:2/0:0);
- LPE(20:5), PC(40:8), PC(18:0/18:1), PC(18:0/18:2), LPE(18:2/0:0);
- PE(16:1e/20:5), PC(18:0/18:1), PC(18:0/18:2), LPE(22:5/0:0), DPA(22:5n-6);
- LPE(20:5), PE(16:1e/20:5), PC(18:0/18:1), PC(18:0/18:2), DPA(22:5n-6);
- LPE(20:5), PE(16:1e/20:5), PC(40:8), PC(18:0/18:2), LPE(22:5/0:0);
- PC(40:8), PC(18:0/18:1), PC(18:0/18:2), LPE(22:5/0:0), DPA(22:5n-6);
- PE(16:1e/20:5), PC(40:8), PC(18:0/18:1), PC(18:0/18:2), LPE(22:5/0:0);
- PE(16:1e/20:5), PC(18:0/18:1), PC(18:0/18:2), DPA(22:5n-6), LPE(18:2/0:0);
- PE(16:1e/20:5), PC(18:0/18:1), PC(18:0/18:2), LPE(22:5/0:0), LPE(18:2/0:0);
- LPE(20:5), PE(16:1e/20:5), PC(18:0/18:2), DPA(22:5n-6), LPE(18:2/0:0);
- PE(16:1e/20:5), PC(40:8), LPE(22:5/0:0), DPA(22:5n-6), LPE(18:2/0:0);
- LPE(20:5), PC(18:0/18:1), PC(18:0/18:2), DPA(22:5n-6), LPE(18:2/0:0);
- LPE(20:5), PC(40:8), PC(18:0/18:1), PC(18:0/18:2), LPE(22:5/0:0);
- LPE(20:5), PE(16:1e/20:5), PC(18:0/18:1), PC(18:0/18:2), LPE(22:5/0:0);
- LPE(20:5), PE(16:1e/20:5), PC(18:0/18:1), LPE(22:5/0:0), LPE(18:2/0:0);
- LPE(20:5), PC(40:8), LPE(22:5/0:0), DPA(22:5n-6), LPE(18:2/0:0);
- LPE(20:5), PE(16:1e/20:5), PC(18:0/18:1), DPA(22:5n-6), LPE(18:2/0:0);
- PE(16:1e/20:5), PC(18:0/18:1), LPE(22:5/0:0), DPA(22:5n-6), LPE(18:2/0:0);
- PE(16:1e/20:5), PC(40:8), PC(18:0/18:1), DPA(22:5n-6), LPE(18:2/0:0);
- PC(40:8), PC(18:0/18:1), LPE(22:5/0:0), DPA(22:5n-6), LPE(18:2/0:0);
- LPE(20:5), PC(40:8), PC(18:0/18:1), LPE(22:5/0:0), LPE(18:2/0:0);
- LPE(20:5), PC(40:8), PC(18:0/18:1), DPA(22:5n-6), LPE(18:2/0:0);
- LPE(20:5), PE(16:1e/20:5), PC(40:8), PC(18:0/18:1), LPE(18:2/0:0);
- LPE(20:5), PC(40:8), PC(18:0/18:1), LPE(22:5/0:0), DPA(22:5n-6);
- LPE(20:5), PE(16:1e/20:5), PC(40:8), PC(18:0/18:1), DPA(22:5n-6);
- LPE(20:5), PE(16:1e/20:5), PC(40:8), PC(18:0/18:1), LPE(22:5/0:0);
- LPE(20:5), PC(18:0/18:1), LPE(22:5/0:0), DPA(22:5n-6), LPE(18:2/0:0);
- LPE(20:5), PE(16:1e/20:5), PC(18:0/18:1), LPE(22:5/0:0), DPA(22:5n-6);
- PE(16:1e/20:5), PC(40:8), PC(18:0/18:1), LPE(22:5/0:0), LPE(18:2/0:0);
- LPE(20:5), PE(16:1e/20:5), PC(40:8), DPA(22:5n-6), LPE(18:2/0:0);
- PE(16:1e/20:5), PC(40:8), PC(18:0/18:1), LPE(22:5/0:0), DPA(22:5n-6); and
- LPE(20:5), PE(16:1e/20:5), PC(40:8), LPE(22:5/0:0), DPA(22:5n-6).

Any combination of six or seven markers are also contemplated as further embodiments of the invention.

In a particular embodiment, the methods of the invention comprise determining all of lipid markers
- Lysoglycerophosphoethanolamine (20:5);
- 1-ether, 2-acylglycerophosphoethanolamine (16:1e/20:5);
- Diacylglycerophosphocholine (40:8);
- Diacylglycerophosphocholine (18:0/18:1);
- Diacylglycerophosphocholine (18:0/18:2);
- Lysoglycerophosphoethanolamine (22:5/0:0);
- Docosapentaenoic acid (22:5n-6); and
- Lysoglycerophosphoethanolamine (18:2/0:0).

In another particular embodiment, the methods of the invention comprise determining lipid markers
- 1-ether, 2-acylglycerophosphoethanolamine (16:1e/20:5); and
- Diacylglycerophosphocholine (40:8).

The predictive method of the invention comprises comparing the level of the markers with a reference value. The comparison of the expression levels of the lipids of interest with the reference value allows determining whether the subject will show a good or poor outcome of IVF. The term "reference value", as used herein, relates to a predetermined criteria used as a reference for evaluating the values or data obtained from the samples collected from a subject. The reference value or reference level can be an absolute value, a relative value, a value that has an upper or a lower limit, a range of values, an average value, a median value, a mean value, or a value as compared to a particular control or baseline value. A reference value can be based on an individual sample value, such as for example, a value obtained from a sample from the subject being tested, but at an earlier point in time. The reference value can be based on a large number of samples, such as from population of subjects of the chronological age matched group, or based on a pool of samples including or excluding the sample to be tested. According to the predictive method of the invention, the level or the expression level of a marker is considered "decreased" when the expression level of said marker in a sample is lower than its reference value. The expression level of a marker is considered to be lower than its reference value when it is at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150%, or more lower than its reference value. For example, a decrease in expression levels below the reference value of at least 0.9-fold, 0.75-fold, 0.2-fold, 0.1-fold, 0.05-fold, 0.025-fold, 0.02-fold, 0.01-fold, 0.005-fold or even less compared with reference value is considered as "decreased" expression level. Likewise, in the context of the predictive method of the invention, the level or the expression level of a marker is considered "increased" when the expression level of said marker in a sample is higher than its reference value. The expression level of a biomarker is considered to be higher than its reference value when it is at least 1.5%, at least 2%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150%, or more higher than its reference value. For example, an increase in expression levels above the reference value of at least 1.1-fold, 1.5-fold, 5-fold, 10-fold, 20-fold, 30-fold, 40-fold, 50-fold, 60-fold, 70-fold, 80-fold, 90-fold, 100-fold or even more compared with the reference value is considered as "increased" expression level.

Methods to determine the expression levels of lipids are well known in the art ̌(Holčapek et al, Lipidomic Analysis, Anal. Chem. 2018, 90, 4249-4257). In a particular embodiment, the expression level of at least one lipid marker is determined by UHPLC-MS. The term "UHPLC -MS" refers to ultra-high performance liquid chromatography coupled with mass spectrometry. In another particular embodiment, the expression level of at least one lipid marker is determined by UHPLC-ToF-MS. The term "UHPLC-ToF-MS" refers to ultra-high performance liquid chromatography coupled with time-of-flight mass spectrometry. A person skilled in the art will know how to perform the technique and identify the corresponding lipid markers. Additional techniques to measure the level of the lipid markers include, without limitation, refractive index spectroscopy (RI), Ultra-Violet spectroscopy (UV), fluorescent analysis, radiochemical analysis, Infrared spectroscopy (IR), Nuclear Magnetic Resonance spectroscopy (NMR), Light Scattering analysis (LS), Mass Spectrometry, Pyrolysis Mass Spectrometry, Nephelometry, Dispersive Raman Spectroscopy, gas chromatography combined with mass spectroscopy, liquid chromatography combined with mass spectroscopy, supercritical fluid chromatography combined with mass spectroscopy, MALDI combined with mass spectroscopy, ion spray spectroscopy combined with mass spectroscopy, capillary electrophoresis combined with mass spectrometry, NMR combined with mass spectrometry and IR combined with mass spectrometry.

In a particular embodiment within the context of the methods of the invention, the subject has undergone a standard IVF cycle. The term "standard IVF cycle" refers to a menstrual mammalian female cycle in which natural ovulation is not allowed to occur. Instead, the eggs are retrieved at the point of maturation and are fertilized in the IVF lab to create embryos. A standard IVF cycle comprises the steps of suppressing the natural menstrual cycle, promoting the egg supply, collecting the oocytes, fertilizing the oocytes and embryo transfer. Typically, the process would require the use of suppressive and stimulatory drugs. Examples of suppressive drugs would include without limitation leuprolide, nafarelin, Antagon or cetrorelix acetate among others. Examples of stimulatory drugs include gonadotropins to stimulate ovaries to produce more follicles.

In another particular embodiment, the endometrial fluid sample is collected as an endometrial fluid aspirate. The term "endometrial fluid aspirate" refers to a fluid produced by the uterine organ forming part of the female genital tract and which has been taken by aspiration, such as vacuum aspiration (i.e., "aspirate sample"). According to the present invention, the aspiration of the fluid is performed without a previous step of saline infusion. That is, the term "aspirate" does not encompass those samples resulting from uterine washings.

In a second aspect the invention relates to a method for selecting a subject for implantation with at least one embryo during IVF, wherein the method comprises determining, in an endometrial fluid sample from the subject, the expression level of at least one lipid marker selected from the group consisting of
- Lysoglycerophosphoethanolamine (20:5);
- 1-ether, 2-acylglycerophosphoethanolamine (16:1e/20:5);
- Diacylglycerophosphocholine (40:8);
- Diacylglycerophosphocholine (18:0/18:1);
- Diacylglycerophosphocholine (18:0/18:2);
- Lysoglycerophosphoethanolamine (22 :5/0:0);
- Docosapentaenoic acid (22:5n-6); and/ or
- Lysoglycerophosphoethanolamine (18:2/0:0),
   wherein
- a decrease in the expression level of Diacylglycerophosphocholine (40:8) and Docosapentaenoic acid (22:5n-6) with respect to a reference value, and
- an increase in the expression level of Lysoglycerophosphoethanolamine (20:5); 1-ether, 2-acylglycerophosphoethanolamine (16:1e/20:5); Diacylglycerophosphocholine (18:0/18:1); Diacylglycerophosphocholine (18:0/18:2); Lysoglycerophosphoethanolamine (22:5/0:0); and Lysoglycerophosphoethanolamine (18:2/0:0) with respect to a reference value is indicative that the subject is selected for implantation with one embryo,
   and wherein
- an increase in the expression level of Diacylglycerophosphocholine (40:8) and Docosapentaenoic acid (22:5n-6) with respect to a reference value, and
- a decrease in the expression level of Lysoglycerophosphoethanolamine (20:5); 1-ether, 2-acylglycerophosphoethanolamine (16:1e/20:5); Diacylglycerophosphocholine (18:0/18:1); Diacylglycerophosphocholine (18:0/18:2); Lysoglycerophosphoethanolamine (22:5/0:0); and Lysoglycerophosphoethanolamine (18:2/0:0) with respect to a reference value
is indicative that the subject is selected for implantation with two or more embryos, or that the embryos are frozen and IVF is postponed.

In this context, in the presence of an adverse lipidomic profile, embryo transfer could be canceled (cryopreserving the embryos) or delayed, or perhaps endometrial fluid could be flushed to wash up the adverse lipids, or even it could be decided to increase the number of embryos to be transferred. On the other hand, if the lipidomic profile is favorable, only single embryo transfer should be performed.

In a third aspect the invention relates to a method for selecting an IVF treatment for a subject, wherein the method comprises determining, in an endometrial fluid sample from the subject, the expression level of at least one lipid marker selected from the group consisting of
- Lysoglycerophosphoethanolamine (20:5);
- 1-ether, 2-acylglycerophosphoethanolamine (16:1e/20:5);
- Diacylglycerophosphocholine (40:8);
- Diacylglycerophosphocholine (18:0/18:1);
- Diacylglycerophosphocholine (18:0/18:2);
- Lysoglycerophosphoethanolamine (22:5/0:0);
- Docosapentaenoic acid (22:5n-6); and/ or
- Lysoglycerophosphoethanolamine (18:2/0:0),
   wherein
- a decrease in the expression level of Diacylglycerophosphocholine (40:8) and Docosapentaenoic acid (22:5n-6) with respect to a reference value, and
- an increase in the expression level of Lysoglycerophosphoethanolamine (20:5); 1-ether, 2-acylglycerophosphoethanolamine (16:1e/20:5); Diacylglycerophosphocholine (18:0/18:1); Diacylglycerophosphocholine (18:0/18:2); Lysoglycerophosphoethanolamine (22:5/0:0); and Lysoglycerophosphoethanolamine (18:2/0:0) with respect to a reference value
   is indicative that an IVF treatment comprising the implantation of one embryo is selected for the subject,
   and wherein
- an increase in the expression level of Diacylglycerophosphocholine (40:8) and Docosapentaenoic acid (22:5n-6) with respect to a reference value, and
- a decrease in the expression level of Lysoglycerophosphoethanolamine (20:5); 1-ether, 2-acylglycerophosphoethanolamine (16:1e/20:5); Diacylglycerophosphocholine (18:0/18:1); Diacylglycerophosphocholine (18:0/18:2); Lysoglycerophosphoethanolamine (22:5/0:0); and Lysoglycerophosphoethanolamine (18:2/0:0) with respect to a reference value
   is indicative that an IVF treatment comprising the implantation of two or more embryos is selected for the subject, or that the embryos are frozen and IVF is postponed.

The term "in vitro fertilization" or "IVF" has been previously defined, and it refers to a process by which oocytes are fertilized by sperm outside of the body, in vitro. IVF is a major treatment in infertility when in vivo conception has failed. The present method may also be performed when the fertilization takes place by "intracytoplasmic sperm injection" or "ICSI", which refers to an in vitro fertilization procedure in which a single sperm is injected directly into an oocyte. This procedure is most commonly used to overcome male infertility factors, although it may also be used where oocytes cannot easily be penetrated by sperm, and occasionally as a method of in vitro fertilization in a number of conditions (poor response, unknown infertility, endometriosis).

The term "embryo" is used herein to refer both to the zygote that is formed when two haploid gametic cells, e.g. an unfertilized secondary oocyte and a sperm cell, unite to form a diploid totipotent cell, e.g. a fertilized ovum, and to the embryo that results from the immediately subsequent cell divisions, i.e. embryonic cleavage, up through the morula, i.e. 16-cell stage and the blastocyst stage (with differentiated trophectoderm and inner cell mass). Thus, the term "embryo" refers to a fertilized oocyte or zygote. Said fertilization may intervene under a classical in vitro fertilization (IVF) or under an intracytoplasmic sperm injection (ICSI) protocol. The term "oocyte" is used herein to refer to an unfertilized female germ cell, or gamete. Oocytes of the subject application may be primary oocytes, in which case they are positioned to go through or are going through meiosis I, or secondary oocytes, in which case they are positioned to go through or are going through meiosis II. By "meiosis" it is meant the cell cycle events that result in the production of gametes. In the first meiotic cell cycle, or meiosis I, a cell's chromosomes are duplicated and partitioned into two daughter cells. These daughter cells then divide in a second meiotic cell cycle, or meiosis II, that is not accompanied by DNA synthesis, resulting in gametes with a haploid number of chromosomes.

The term "implanted" or "implantation" has been previously defined and it is used to describe the process of attachment and invasion of the uterus endometrium by the blastocyst in placental animals. The term "blastocyst" is used herein to describe all embryos that reach cavitation (i.e., the formation of cavities).

In a fourth aspect, the invention relates to the use of reagents specific for the determination of the expression levels of
- Lysoglycerophosphoethanolamine (20:5);
- 1-ether, 2-acylglycerophosphoethanolamine (16:1e/20:5);
- Diacylglycerophosphocholine (40:8);
- Diacylglycerophosphocholine (18:0/18:1);
- Diacylglycerophosphocholine (18:0/18:2);
- Lysoglycerophosphoethanolamine (22:5/0:0);
- Docosapentaenoic acid (22:5n-6); and/ or
- Lysoglycerophosphoethanolamine (18:2/0:0),
for predicting the outcome of, or assessing the chances of success of, IVF in a subject.

In a fifth aspect the invention relates to an endometrial fluid wash solution for use in the treatment of infertility in a subject, wherein the wash solution is suitable for
- decreasing the expression level of Diacylglycerophosphocholine (40:8) and Docosapentaenoic acid (22:5n-6) with respect to a reference value, and
- increasing the expression level of Lysoglycerophosphoethanolamine (20:5); 1-ether, 2-acylglycerophosphoethanolamine (16:1e/20:5); Diacylglycerophosphocholine (18:0/18:1); Diacylglycerophosphocholine (18:0/18:2); Lysoglycerophosphoethanolamine (22:5/0:0); and Lysoglycerophosphoethanolamine (18:2/0:0) with respect to a reference value

In mammalian females, the procedure of washing the uterine cavity has been commonly used with the main objective to set non-invasive prenatal diagnosis, innovative with respect to invasive traditional methods of amniocentesis and chorionic villus sampling. In recent years, such technique has been applied to evaluate the conditions of the uterine cavity before the transfer of the embryo in the cycles of assisted reproduction. The application of this procedure consists in the insertion into the uterus of a buffer solution with the aim at collecting uterine secretes and evaluate their organic and inorganic biological components. It has the purpose to identify possible markers, predictive of implantation success. In addition, the uterine washing technique provides a non-invasive practice that allows restoration of the uterine physiological conditions through mechanical removal of secretes, which may alter the implantation conditions and/or to provide essential compounds for embryo implantation. Preferably, said composition suitable for topical administration through uterine washing essentially consists in a physiological saline solution. Said physiological saline solution would enable the removing of the elevated lipids which prevent a successful embryo implantation in an IVF treatment such as Diacylglycerophosphocholine (40:8) and Docosapentaenoic acid (22:5n-6). Further, the washing solution may be formulated to restore the lipids markers that favour or enable a successful embryo implantation, such as Lysoglycerophosphoethanolamine (20:5); 1-ether, 2-acylglycerophosphoethanolamine (16:1e/20:5); Diacylglycerophosphocholine (18:0/18:1); Diacylglycerophosphocholine (18:0/18:2); Lysoglycerophosphoethanolamine (22:5/0:0); and Lysoglycerophosphoethanolamine (18:2/0:0). Thus, in a particular embodiment the endometrial fluid wash solution comprises an effective amount of
- Lysoglycerophosphoethanolamine (20:5);
- 1-ether, 2-acylglycerophosphoethanolamine (16:1e/20:5);
- Diacylglycerophosphocholine (40:8);
- Diacylglycerophosphocholine (18:0/18:1);
- Diacylglycerophosphocholine (18:0/18:2);
- Lysoglycerophosphoethanolamine (22:5/0:0);
- Docosapentaenoic acid (22:5n-6); and/ or
- Lysoglycerophosphoethanolamine (18:2/0:0).

In addition the washing solution may comprise essential and non-essential amino acids, and buffer salts. Preferably said buffer salts are salts of calcium, sodium, potassium and magnesium, and mixtures thereof. The use of buffer salts and amino acids is provided for producing a formulation with a pH comprised between 7 and 8 (preferably between 7.5-7.8), to replicate the micro- environment suitable for the embryo (and similar to that of culture media in which the embryo grows before the transfer into the uterus) .

According to an alternative embodiment, the compositions of the invention may be formulated as gel suitable for uterine administration, more precisely for in situ administration in the uterine cavity, preferably with a medical device being an intrauterine "T shaped" device. It is also possible to foresee controlled release gel formulations for mucosal administration.

Considering that the uterine washing technique as above described revealed to be a non-invasive practice and that allows restoration of the uterine physiological conditions through mechanical removal of secretes, which may alter the implantation conditions, the invention has as a further object a medical device for uterine washing comprising a sterile container pre-filled or to be filled with a composition as above defined.

More precisely, the device according to the present invention can be filled at the moment of use with a sterile solution suitable for the uterine or endometrial washing. According to a preferred embodiment the sterile container is disposable. More preferably the sterile container (preferably a disposable sterile container) is pre-filled with physiological solution supplemented with the required lipids, buffer salts and amino acids such as to produce a formulation with pH comprised between 7-8, preferably between 7.5 and 7.8.

The uterine washing shall be performed with the aid of a catheter after pick-up with echographic guide. The next embryos transfer or implantation shall take place averagely three days after the washing (1-5 days), and no later than 6 days of embryo development.

Finally, in a sixth aspect the invention relates to an endometrial fluid wash solution comprising an effective amount of
- Lysoglycerophosphoethanolamine (20:5);
- 1-ether, 2-acylglycerophosphoethanolamine (16:1e/20:5);
- Diacylglycerophosphocholine (40:8);
- Diacylglycerophosphocholine (18:0/18:1);
- Diacylglycerophosphocholine (18:0/18:2);
- Lysoglycerophosphoethanolamine (22:5/0:0);
- Docosapentaenoic acid (22:5n-6); and/ or
- Lysoglycerophosphoethanolamine (18:2/0:0).

In a particular embodiment, the lipids of the invention are the only lipids present in the endometrial fluid wash solution.

All the terms and embodiments described herein are equally applicable to all aspects of the invention. The invention will be further described by way of the following examples, which are to be considered as merely illustrative and not limitative of the scope of the invention.

### EXAMPLES

### Example 1: Materials and methods

### Ethical approval

This study was approved by the Institutional Review Board (code CEIC 11/45). All the patients included in the study provided informed consent.

### Study population

The population under study consisted of 29 women undergoing an IVF/intracytoplasmic sperm injection (ICSI) cycle at the Reproductive Unit of Cruces University Hospital, in which EFA was obtained at the time of embryo transfer. Out of the 29 women, 15 achieved pregnancy (implantative endometrium group) and 14 did not (non-implantative endometrium group).

The inclusion criteria were: (i) first or second IVF/ICSI cycle; (ii) woman's age between 18 and 40 years; (iii) no known uterine or endometrial conditions (myomas, polyps, malformations, adenomyosis, endometritis, thin endometrium); (iv) absence of hydrosalpinx; (v) no infectious risk; (vi) transfer on day three of 1-2 fresh embryos with at least one of good quality; and (vii) fresh own oocyte cycles without preimplantation genetic diagnosis or testicular biopsy. Women in whom an abnormal cervical mucus or leucorrhea was observed during the collection of the endometrial fluid sample were excluded from the study.

The characteristics of the study population are detailed in Table 1 below. The most common indications for IVF were: male factor, insemination failure, tubal factor and endometriosis.

**Table 1. Demographic and clinical characteristics in implantative and non-implantative cycles.**

| | **Implantative cycles (*n* = 15)** | **Non-implantative cycles (*n* = 14)** |
|---|---|---|
| Age, years | 36.2 ± 2.6 | 36.6 ± 2.8 |
| Body mass index, kg/m² | 25.4 ± 3.0 | 25.9 ± 3.2 |
| % Smokers | 26.7 | 28.6 |
| % Male factor | 40 | 35.7 |
| % Previous insemination failure | 20 | 21.4 |
| % Tubal factor | 6.7 | 14.3 |
| % Endometriosis | 13.3 | 7.1 |
| % First cycle | 60 | 50 |
| Days of stimulation | 11.9 ± 1.9 | 11.6 ± 1.7 |
| Estradiol the day of hCG (pg/ml) | 2104.6 ± 928.1 | 2031.2 ± 1048.5 |
| P4 the day of hCG (ng/ml) | 0.84 ± 0.22 | 0.95 ± 0.23 |
| Oocytes obtained | 10.3 ± 6.7 | 10.8 ± 7.3 |
| MII oocytes | 7.9 ± 6.2 | 8.2 ± 5.4 |
| Embryos obtained | 5.3 ± 4.8 | 4.8 ± 4.7 |
| Embryos transferred | 1.4 ± 0.4 | 1.6 ± 0.3 |
| % Twins | 13.3 | |

### Sample collection

The standard IVF protocol used in the context of the present invention has been described previously (Matorras, et al., 2009, Agirregoitia, et al., 2016). Briefly, it consisted in either a long agonist protocol or a conventional antagonist protocol, ovarian stimulation being performed with only recombinant follicle-stimulating hormone (rFSH) in women ≤35 years, and with combinations of rFSH and human menopausal gonadotropin (hMG) or luteinizing hormone (LH) in women aged 36-39. Ovulation was triggered with recombinant human chorionic gonadotropin (rec hCG) (Ovitrelle, Merck, Madrid, Spain) when ≥3 follicles ≥18.5 mm were observed. Oocyte retrieval was scheduled 36 h after triggering ovulation. If on the day of administering hCG, the progesterone level was >1.6 ng/mL, embryos were frozen and transfer was performed in a later cycle (and the case was not included in our study). The luteal phase was supported by progesterone 200 mg every 12 h, starting the day of oocyte pick-up. The hormone was administered orally until embryo transfer, and thereafter vaginally. Pregnancy was defined as visualization of a gestational sac four weeks after embryo transfer. Biochemical pregnancies were not included in the study.

Women underwent a standard IVF cycle, and a sample of endometrial fluid was taken immediately before embryo transfer. This sample was obtained using an embryo transfer catheter connected to a 10 mL syringe, under ultrasound guidance. The catheter was inserted gently, avoiding traumatizing the cervix or touching the uterine fundus. We excluded women in whom we observed abnormal cervical mucus or leucorrhea. Aspiration was performed manually. Only samples showing no presence of blood were considered for lipidomic analysis. Collected samples were stored at -80 °C until analysis.

### Lipid extraction and UHPLC-MS analysis

The extraction method was set up using a combination of organic solvents that allows a wide coverage of the lipidome of the endometrial fluid. Extracts were analyzed in an ultra-high performance liquid chromatography coupled to time-of-flight mass spectrometry (UHPLC-ToF-MS) based analytical platform, established for an optimal profiling of lipids. Briefly, the endometrial fluid samples were defrosted on ice and were mixed with 450 µL of water / methanol / chloroform (1:2.5:1, v/v/v). After centrifugation at 16,000 x g for 20 minutes at room temperature, 200 µL of supernatants were collected, dried, reconstituted in 60 µL of methanol for the UHPLC-ToF-MS profiling of fatty acids (FA), oxidized FA, N-acyl ethanolamines, bile acids and lysoglycerophospholipids (lysophosphatidylcholines, lysophosphatidylethanolamines and lysophosphatidylinositols, either acyl- or ether-linked species). In addition, other 200 µL of supernatants were collected and mixed with sodium chloride (50 mM). The extracts were incubated for 30 minutes at -20 °C. After centrifugation at 16,000 x g for 15 minutes at 4 °C, 35 µL of the organic phase was collected and the solvent removed. The dried extracts were then reconstituted in 60 µL of acetonitrile / isopropanol (1:1, v/v) for the analysis of glycerolipids (triglycerides and diglycerides), cholesteryl esters, sphingolipids (ceramides, hexosylceramides and sphingomyelins) and glycerophospholipids (phosphatidylcholines, phosphatidylethanolamines and phosphatidylinositols, either diacyl or 1-ether, 2-acyl-linked species).

Lipidomic profiles of the endometrial fluid aspirates were semi-quantified as previously described (Manni 2017, Barr et al., 2012) with two separate UHPLC-ToF-MS based platforms. Chromatographic separation and mass spectrometric detection conditions are detailed elsewhere herein. For both analytical platforms, randomized sample injections were performed. The study samples were accompanied by repeat extracts of a pooled sample, used for the assessment of the data quality. This pooled sample was analyzed at the same time as the individual samples, evenly distributed over the batch.

Lipid extracts were analyzed by ultra-high performance liquid chromatography coupled to time-of-flight mass spectrometry (UHPLC-ToF-MS). Targeted lipidomic profiles were UHPLC-ToF-MS based analytical platforms, established for an optimal profiling of the lipidome of endometrial fluid aspirates. First platform (Platform 1) is optimized for the lipidomic profiling of fatty acids, oxidized fatty acids, N-acyl ethanolamines, bile acids and lysoglycerophospholipids, while the Platform 2 covers glycerolipids (triglycerides and diglycerides), cholesteryl esters, sphingolipids (ceramides, hexosylceramides and sphingomyelins) and glycerophospholipids.

Chromatographic separation and mass spectrometric detection conditions employed for each platform are summarized in Table 2.

Data were acquired at a resolution of 12000 and 20000 for Platform 1 [acquisition over a range of mass to charge ratio (m/z) of 50-1000] and Platform 2 (range of m/z 50-1200) respectively, using an accumulation time of 0.2 s per spectrum. All spectra were mass-corrected in real time by reference to leucine enkephalin, infused at a constant flow rate of 50 and 10 µL / min (external pump) in Platform 1 and 2, respectively, through an independent reference electrospray. The frequency was set at 10 s. A single lock mass calibration at m/z 556.2771 in positive ion mode (Platform 2) and m/z 554.2615 in negative ion mode (Platform 1) was used for the complete analysis. The system was controlled by Masslynx 4.1 (Waters Corp., Mildford, MA). The overall quality of the analysis procedure was monitored using five repeat injections of a pooled sample, considered as the quality control sample. Generally, the retention time (Rt) stability was <6 s injection-to-injection variation and the mass accuracy <3 ppm for m/z 400-1200, and <1.2 mDa for m/z 50-400. The mean coefficients of variation, CV, in the quality control samples was CV = 5.31% for the ion features detected in the Platform 1; CV = 4.32% for ion features detected in Platform 2; and CV = 4.69% considering all the metabolites reported in this work.

**Table 2. Chromatographic separation and mass spectrometric detection conditions employed per analytical platform**

| | **UHPLC-MS for Lipidomics Platform 1** | **UHPLC-MS for Lipidomics Platform 2** |
|---|---|---|
| **System** | UHPLC-LCT XE Premier | UHPLC-Xevo G2 |
| **Column type** | UPLC BEH C18, 2.1 x 100 mm, 1.7 µm | UPLC BEH C18, 2.1 x 100 mm, 1.7 µm |
| **Flow rate** | 0.14 mL / min | 0.40 mL / min |
| **Solvent A** | H2O + 0.05% formic acid | H2O + ACN + 10mM ammonium formate |
| **Solvent B** | ACN + 0.05% formic acid | ACN + Isopropanol + 10mM ammonium formate |
| **(%B), time** | 0%, 0 min | 40%, 0 min |
| **(%B), time** | 50%, 2 min | 100%, 10 min |
| **(%B), time** | 100%, 13 min | 40%, 15 min |
| **Column temperature** | 40 °C | 60 °C |
| **Injection volume** | 2 µL | 3 µL |
| **Ionization** | ESI -ve | ESI +ve |
| **Source temperature** | 120 °C | 120 °C |
| **Nebulization N₂ flow** | 600 L / h | 1000 L / h |
| **Nebulization N₂ temperature** | 350 °C | 500 °C |
| **Cone N₂ flow** | 30 L / h | 30 L / h |
| **Capillary voltage** | 2.8 kV | 3.2 kV |
| **Cone voltage** | 50 V | 30 V |

### Metabolite Identification

Online tandem mass spectrometry (MS/MS) experiments for metabolite identification were performed on a Waters QTOF Premier and Xevo G2 (Waters Corp., Milford, MA) instruments operating in both the positive and negative ion electrospray modes. Source parameters were identical to those employed in the profiling experiments, except for the cone voltage which was increased (30-70 V) when pseudo MS/MS/MS data was required, as described in detail previously (Barr 2010).

LC-MS features (as defined by retention time, mass-to-charge ratio pairs; Rt-m/z), were associated with fatty acids (FA), N-acylethanolamines (NAE) and oxidized fatty acids by comparison of their accurate mass spectra and chromatographic retention times in the extracts with those obtained using available reference standards. A metabolic feature with m/z value between 400 and 1000 Da was considered unambiguously identified when Rt difference with respect to the standard was smaller than 3 s and the deviation from its m/z value (δm/z) smaller than 3 ppm. For metabolites with m/z values smaller than 400 Da, the criterion followed with respect to Rt was the same, but setting the δm/z limit to 1.2 mDa. The ion features considered for the following data analysis were the adducts [M-CO₂H]⁻, [M-CO₂H]⁻ and [M-H]⁻ for FA, NAE and oxidized FA, respectively. The nomenclature C:Dn-x is used for these fatty acyl species, where C is the number of carbon atoms, and D is the number of double bonds in the fatty acid chains. A double bond is located on the xth carbon-carbon union, counting from the terminal methyl group towards the carbonyl carbon. However, since the physiological properties of unsaturated fatty acids largely depend on the position of the first unsaturation relative to the end position and not the carboxylate, the location is signified by ω or "n" minus the unsaturation location, i.e., 20:5 (n-3). An "x" in the name of some FA indicates the unknown position the double bounds, as reference standards for full identification were not available.

For diglycerides (DG), triglycerides (TG), cholesteryl esters (ChoE), glycerophosphatidylcholines (PC), glycerophosphatidylethanolamines (PE), glycerophosphatidylinositols (PI), sphingomyelins (SM), ceramides (Cer), and monohexosylceramides (CMH) species, a theoretical m/z database was first generated for all possible combinations of fatty acid derived moieties. The association of detected Rt-m/z pairs with lipid species contained in the theoretical database was subsequently established either by comparison of their accurate mass spectra and chromatographic retention times with those obtained using available reference standards or, where these were not available, by accurate mass MS/MS fragment ion analysis, as described in detail previously (Barr 2010, Murphy, R C, Fiedler J, Hevko J. 2001.. Analysis of Nonvolatile Lipids by Mass Spectrometry. Chem. Rev. 101: 479-526). Briefly, the regiochemistry for glycerophospholipids was determined as follows. Monoacylglycerophosphatidylcholine regiochemistry was determined by the fragmentation behavior of the corresponding [M+Na]⁺ ions, as observed in their positive ion mode MS/MS spectra. This followed previous reports that have shown that spectra of sn-1 monoacyl species show a high m/z= 104.1070 (choline)/ 146.9818 (sodiated cyclic ethylene phosphate) product ion ratio from the [M+Na]⁺ ion. The converse situation is observed in the case of the sn-2 monoacyl species (Han 1996). The regiochemistry in phosphatidylethanolamines was determined considering that the sn-2 carboxylate ion should be much more intense than sn1 carboxylate ion.

The ion features considered for the following data analysis of these lipid classes were the adducts [M-CO₂H]⁻ for monoacyl- and monoether-PC; [M-H]⁻ for monoacyl- and monoether-PE and PI; [M+H]⁺ for diacyl- or 1-ether,2-acyl-PC, PE and PI, CMH and SM; [M+H-H₂O]⁺ for Cer; [M+Na]⁺ for DG; [M+NH₄]⁺ for ChoE and TG. The nomenclature for these lipid species follows the rules:
- Glycerolipids species (DG and TG): C:D nomenclature is used, where C is the number of carbon atoms and D is the number of double bonds considering all the acyl chains esterified to glycerol. The position of the double bonds in the acyl chains is not considered, as well as the position of the acyl chains.
- ChoE: C:D nomenclature is used, where C is the number of carbon atoms and D is the number of double bonds in the acyl chains esterified to the cholesterol. The position of the double bonds in the acyl chains is not considered.
- Sphingolipids species (SM, Cer and CMH): sA:B/C:D nomenclature is used, where sA:B represents the sphingoid base: s18:1, sphingosine; s18:2, sphingadiene; s18:0, sphinganine. C:D indicates the number of carbon atoms C, and double bonds D, contained in the N-linked fatty acid. The position of the double bonds in the acyl chains is not considered.
- Glycerophospholipids (PC, PE, and PI) as diacyl, 1-acyl, 2-acyl, 1-ether or 1-ether, 2acyl-linked species: A:B/C:D nomenclature is used, where A:B and C:D refer to the number of carbon atoms and number of double bonds contained in the sn-1 and sn-2 side chains, respectively. For glycerophospholipids containing an ether moiety the prefix, O-, denotes the presence of an alkyl ether (plasmanyl) substituent, i.e., PC(O-16:0/16:0), whereas the prefix P- refers to a vinyl ether (alkenyl or plasmenyl) substituent, i.e., PC(P-16:0/16:0). The suffix, e, indicates the presence of an ether linked substituent, although plasmanyl or plasmenyl classification has not been confirmed. X:Y nomenclature (where X = A+C and Y = B+D) is used where evidence was found for the contribution of multiple species to a single chromatographic peak. The position of the double bonds in the acyl chains is not considered.

### Data Analysis

The steps followed for data analysis are summarized in Figure 1. First, data pre-processing was carried out using the TargetLynx application manager for MassLynx 4.1 software (Waters Corp., Milford, USA). The LC-MS features (as defined by retention time and mass-to-charge ratio pairs, Rt-m/z) included in this study were identified prior to the analysis, either by comparison of their accurate mass spectra and chromatographic Rt with those of available reference standards or, where these were not available, by accurate mass MS/MS fragment ion analysis, as described elsewhere herein. Lipid nomenclature and classification follows the LIPID MAPS convention, www.lipidmaps.org.

Detected LC-MS features were included in the data pre-processing step. Data normalization was performed following the procedure described by Manni et al. (Manni et al 2017). The peak intensities for each ion feature included in the analysis were normalized to the sum of the peak intensities within each sample. There was no significant difference (p-value > 0.05) between the sum of the peak intensities for the groups being compared in this study (implantative vs. non-implantative endometrium). Missing values were evaluated in the data matrix. In this study, the missing values are due to observations below the sensitivity threshold. One tenth of the minimum value was assigned to these observations (Hrydziuszko et al., 2012; Hair et al., 2009; Baraldi et al., 2010). Then, univariate data analysis was performed. Data in groups are represented as means ± SDM. Metabolites were evaluated by calculating group percentage changes and unpaired Student's t-test p-value or Welch test where the variances were not equal. All calculations were performed using the statistical software package R v.3.4.1 (R Development Core Team, 2017; http://cran.r-project.org) (Wickham, 2011).

Once the univariate data analysis was performed, an exploratory data modeling was carried out including the significant metabolites (p < 0.05). Several model methodologies were tested: Decision Trees, Linear Classifiers, Neuronal Network, Random Forest, K-Nearest Neighbors and Support Vector Machine (SVM) (Figure 1). All these methodologies were also tested including all detected variables (a total of 294 lipids). A detailed description is included elsewhere herein. Furthermore, a pre-processing of the data was also evaluated: no pre-processing data, centered and scaled, Box-Cox (Box, G. E. P. and Cox, D. R., 1964) and Box-Cox and centered and scaled. These transformation techniques are usually applied to omics data (Robert A van den Berg et al., 2006; Yang et al., 2015).

The model providing the best results in the exploratory data analysis was subsequently improved and evaluated applying rigorous cross validation techniques and pre-process methodologies to the data. A three-fold Cross Validation was performed with a 10 resampling for each parameter (or tuple of parameters). The caret R-package was used to evaluate this methodology (Kuhn et al., 2018, Kuhn et al., 2013).

Receiver Operating Characteristics (ROC) curve analysis was used to assess the discriminatory power of the model. ROC graphs are useful for organizing classifiers and visualizing their performance. This analysis is commonly used in medical decision making, and in recent years have been used increasingly in machine learning and data mining research (Fawcett, 2006). Overall diagnostic accuracy of the model was evaluated by the area under the ROC curve (AUC) with its associated standard error. Sensitivity, specificity, Positive Predictive Value (PPV) and Negative Predictive Value (NPV), were also calculated as described elsewhere herein. Additionally, the likelihood ratios were used for assessing the performance of a diagnostic test: Positive Likelihood Ratio (PLR) and Negative Likelihood Ratio (NLR).

### Data Modeling

Several model methodologies were tested:
- Decision Trees: The decision trees build classification or regression models in the form of a tree structure. It breaks down a data set into smaller and smaller subsets while at the same time an associated decision tree is incrementally developed. The final result is a tree with decision nodes and leaf nodes. Among the different decision trees methodologies, we applied in this study: treebag, Bagged CART without tuning parameters; rpart, a CART model with *Complexity Parameter* as tuning parameter; J48, like a C4.5 tree with *Confidence Threshold* and *Minimum Instances Per Leaf* as tuning parameters.
- Linear Classifiers: A linear classifier is a method that achieves a classification based in a linear combination of the variables (metabolites). Although multiple linear classifiers have been used elsewhere, we applied Naïve-Bayes, Generalized Linear Models and Flexible Discriminant Analysis with *degree* and *nprune* as tuning parameters.
- Neuronal Network: A neural network consists of units (neurons), arranged in layers, which convert an input vector into some output. Each unit takes an input, applies a (often nonlinear) function to it and then passes the output on to the next layer. The *size* (hidden units) and *decay* (weight decay) parameters were used as tuning parameters.

- Random Forest: Random forests or random decision forests are an ensemble learning method for classification (and regression) and other tasks, that operate by constructing a multitude of decision trees at training time and outputting the class that is the mode of the classes (classification) of the individual trees.
- Support Vector Machine: Support Vector Machines (SVM) are based on the concept of decision planes that define decision boundaries. A decision plane is one that separates between a set of objects having different class memberships. There are several kernels to construct a SVM. We applied the most common kernels in this study: Linear, Radial and Polynomial, each of them with their specific tuning parameters: Linear (Cost), Radial (Cost and Sigma), Polynomial (Cost, Scale and Polynomial degree).

### ROC Analysis

Receiver Operating Characteristics (ROC) graphs are useful for organizing classifiers and visualizing their performance. ROC graphs are commonly used in medical decision making, and in recent years have been used increasingly in machine learning and data mining research.

Let *Y* and *Y'* be random variables representing the class and the prediction for a randomly drawn sample, respectively. Following the notation of the ROCR package in R, the inventors denote by + and - the positive and negative class, respectively. Further, the inventors use the following abbreviations for empirical quantities: P (# positive samples), N (# negative samples), TP (# true positives), TN (# true negatives), FP (# false positives), FN (# false negatives).

With these definitions we calculate the following parameters:
- Sensitivity: P(Y' = + | Y = +). Estimated as: TP/P.
- Specificity: P(Y' = - | Y = -). Estimated as TN/N.
- Positive Predictive Value: P(Y = + | Yhat = +). Estimated as: TP/(TP+FP).
- Negative Predictive Value: P(Y = - | Yhat = -). Estimated as: TN/(TN+FN).
- Accuracy: P(Y' = Y). Estimated as: (TP + TN)/(P + N).

### Example 2: Results

The inventors analyzed the lipidome of EFA samples collected from 15 women that achieved pregnancy and 14 that did not. The inventors used an UHPLC-ToF-MS analytical platform able to detect a total of 294 different lipid species, including FA (24), oxidized FA (5), N-acyl ethanolamines (2), bile acids (5), steroids (1), cholesterol, cholesteryl esters (8), diglycerides (7), triglycerides (48), phosphatidylcholines (106), phosphatidylethanolamines (47), phosphatidylinositols (4), ceramides (11), monohexosylceramides (2) and sphingomyelins (23). Evaluation of the data revealed 174 missing values (< 2%) due to levels below the limits of detection. Missing values were randomly distributed in implantative and non-implantative groups, and the percentage of missing values remained lower than 7% for all the metabolites.

### Differential lipid levels between implantative and non-implantative endometrial fluids

Univariate assessment of the 294 lipids for discriminating between implantative and non-implantative EFA was performed by calculating group percentage changes and unpaired Student's t-test p-value or Welch test where the variances were not equal (Figure 2). Eight lipids were significantly altered in the non-implantative condition under the t-test criterion (p < 0.05) (Table 2). Except the polyunsaturated fatty acid (PUFA) docosapentaenoic acid (22:5n-6), all of them were glycerophospholipids (phosphatidylcholines and phosphatidylethanolamines) with unsaturated acyl chains. Only two of these metabolites increased in the non-implantative groups, the polyunsaturated phosphatidylcholine PC(40:8) and the omega-6 docosapentaenoic acid (Table 3).

**Table 3. Significantly (p<0.05) altered metabolites in non-implantative comparing to implantative groups. Fold-changes and unpaired Student's t-test p-values (or Welch test where the variances were not equal) are indicated.**

| **Metabolite** | **Metabolic class** | **Fold-change** | **p-value** |
|---|---|---|---|
| LPE(20:5) | Lysoglycerophosphoethanolamine | 0.416 | 0.010 |
| PE(16:1e/20:5) | 1-ether, 2-acylglycerophosphoethanolamine | 0.636 | 0.014 |
| PC(40:8) | Diacylglycerophosphocholine | 1.585 | 0.027 |
| PC(18:0/18:1) | Diacylglycerophosphocholine | 0.751 | 0.031 |
| PC(18:0/18:2) | Diacylglycerophosphocholine | 0.769 | 0.035 |
| LPE(22:5/0:0) | Lysoglycerophosphoethanolamine | 0.562 | 0.037 |
| Docosapentaenoic acid (22:5n-6) | Omega 6 - Polyunsaturated fatty acid | 1.611 | 0.048 |
| LPE(18:2/0:0) | Lysoglycerophosphoethanolamine | 0.623 | 0.048 |

### Predictive model for implantative endometrial status

Different methodologies (Decision Trees, Linear Classifiers, Neuronal Network, Random Forest, K-Nearest Neighbors and SVM) and pre-processing approaches (no pre-processing data, centered and scaled, Box-Cox, and Box-Cox and centered and scaled) were evaluated in order to generate a predictive model of the endometrial implantative status. These methodologies were tested including all the detected lipids and only with the eight metabolites selected based on the univariate analysis (Table 3). The discriminatory power of the models was assessed using ROC curve analysis (Figure 3). Despite different pre-processing approaches were tested, similar results were obtained. Therefore, we select the approach without pre-processing for simplicity. A summary of the performance of all the models without a previous pre-processing is shown in Figure 4, representing the AUC, sensitivity and specificity distribution in the cross-validation process.

Independently of the methodology, the performance of all the models decreased when all the detected lipids were considered if compared to the models performed only with the selected metabolites based on the t-test criterion (Figure 4), and therefore, the inclusion of all the metabolites was no further considered. Assessment of the discriminatory power of models with the best eight metabolites was then performed, selecting SVM algorithms as the best choice for classifying the endometrial implantative status. The most common kernels to construct SVM models were also considered (linear, radial and polynomial; each of them with an optimization of their specific tuning parameters), obtaining similar results in the ROC curve analysis (Table 4 and Figure 5). The inventors thus propose as final model the SVM with a linear kernel without pre-processing the data, as it allows for a greater parsimony of model design achieving a high efficiency of parameterization. The cost, or the tuning parameter C, was optimized to C = 0.28. The diagnostic performances of this model, showed an AUC, sensitivity, specificity, PPV and NPV of 0.893±0.07, 0.857, 0.800, 0.800 and 0.857, respectively.

**Table 4. ROC analysis summary of Support Vector Machine models depending on the applied kernel without pre-processing of the data. Analysis performed for the optimal tuning parameters per kernel (Linear kernel: C = 0.28; Radial kernel: C = 2.74 and sigma = 0.017; Polynomial kernel: C = 1.09, scale = 0.024 and degree = 1).**

| | **Linear** | **Radial** | **Polynomial** |
|---|---|---|---|
| Accuracy | 0.828 | 0.862 | 0.828 |
| Sensitivity | 0.857 | 0.857 | 0.857 |
| Specificity | 0.800 | 0.867 | 0.800 |
| Positive Predictive Value (PPV) | 0.800 | 0.857 | 0.800 |
| Negative Predictive Value (NPV) | 0.857 | 0.867 | 0.857 |
| Positive Likelihood Ratio (PLR) | 4.286 | 6.429 | 4.286 |
| Negative Likelihood Ratio (NLR) | 0.179 | 0.165 | 0.179 |

### Example 3: Reduced signatures

Having determined the optimal set of lipid markers, the inventors evaluated whether the individual components of the lipid signature or reduced combination of components of the lipid signature may also have predictive value in order to predict the outcome of, or assess the chances of success of, in vitro fertilisation (IVF) in a subject. An SVM model with linear kernel was generated for each combination of markers (best model found in the study). The validation process involved dividing the population into training (75%) and test (25%) 1000 times. The predictive value is represented by AUC values. The AUC value presented is the average value of the thousand iterations in training and in tests.

**Individual components**

| **Metabolite** | **Average AUC Train** | **Average AUC test** |
|---|---|---|
| LPE(20:5) | 0.7321 | 0.7365 |
| PE(16:1e/20:5) | 0.7595 | 0.7602 |
| PC(40:8) | 0.7473 | 0.7459 |
| PC(18:0/18:1) | 0.7232 | 0.7179 |
| PC(18:0/18:2) | 0.7553 | 0.7516 |
| LPE(22:5/0:0) | 0.6766 | 0.6863 |
| DPA(22:5n-6) | 0.6930 | 0.6834 |
| LPE(18:2/0:0) | 0.6896 | 0.6902 |

**Combinations of two metabolites**

| **Metabolite** | **AUC train** | **AUC test** |
|---|---|---|
| LPE(20:5) and DPA(22:5n-6) | 0.7858 | 0.7761 |
| LPE(20:5) and PE(16:1e/20:5) | 0.7875 | 0.7718 |
| LPE(20:5) and PC(40:8) | 0.7864 | 0.7768 |
| LPE(20:5) and PC(18:0/18:2) | 0.7621 | 0.7646 |
| LPE(20:5) and LPE(22:5/0:0) | 0.7594 | 0.7625 |
| LPE(20:5) and LPE(18:2/0:0) | 0.7627 | 0.7627 |
| LPE(20:5) and PC(18:0/18:1) | 0.7575 | 0.7500 |
| PE(16:1e/20:5) and PC(40:8) | 0.9228 | 0.8928 |
| PE(16:1e/20:5) and PC(18:0/18:2) | 0.7623 | 0.7507 |
| PE(16:1e/20:5) and DPA(22:5n-6) | 0.7605 | 0.7689 |
| PE(16:1e/20:5) and LPE(18:2/0:0) | 0.7615 | 0.7697 |
| PE(16:1e/20:5) and LPE(20:5) | 0.7629 | 0.7606 |
| PE(16:1e/20:5) and LPE(22:5/0:0) | 0.7628 | 0.7643 |
| PE(16:1e/20:5) and PC(18:0/18:1) | 0.7560 | 0.7549 |
| PC(40:8) and PE(16:1e/20:5) | 0.9246 | 0.8845 |
| PC(40:8) and PC(18:0/18:2) | 0.7598 | 0.7584 |
| PC(40:8) and LPE(20:5) | 0.7585 | 0.7675 |
| PC(40:8) and LPE(22:5/0:0) | 0.7583 | 0.7654 |
| PC(40:8) and LPE(18:2/0:0) | 0.7669 | 0.7616 |
| PC(40:8) and DPA(22:5n-6) | 0.7636 | 0.7637 |
| PC(40:8) and PC(18:0/18:1) | 0.7564 | 0.7603 |
| PC(18:0/18:1) and PC(18:0/18:2) | 0.7537 | 0.7477 |
| PC(18:0/18:1) and PC(40:8) | 0.7507 | 0.7587 |
| PC(18:0/18:1) and DPA(22:5n-6) | 0.7577 | 0.7499 |
| PC(18:0/18:1) and LPE(18:2/0:0) | 0.7577 | 0.7551 |
| PC(18:0/18:1) and PE(16:1e/20:5) | 0.7586 | 0.7608 |
| PC(18:0/18:1) and LPE(22:5/0:0) | 0.7561 | 0.7613 |
| PC(18:0/18:1) and LPE(20:5) | 0.7575 | 0.7583 |
| PC(18:0/18:2) and PC(40:8) | 0.7564 | 0.7559 |
| PC(18:0/18:2) and LPE(20:5) | 0.7581 | 0.7637 |
| PC(18:0/18:2) and PE(16:1e/20:5) | 0.7610 | 0.7620 |
| PC(18:0/18:2) and LPE(22:5/0:0) | 0.7574 | 0.7635 |
| PC(18:0/18:2) and PC(18:0/18:1) | 0.7547 | 0.7629 |
| PC(18:0/18:2) and DPA(22:5n-6) | 0.7580 | 0.7523 |
| PC(18:0/18:2) and LPE(18:2/0:0) | 0.7600 | 0.7471 |
| LPE(22:5/0:0) and PC(18:0/18:2) | 0.7556 | 0.7459 |
| LPE(22:5/0:0) and PE(16:1 e/20:5) | 0.7572 | 0.7626 |
| LPE(22:5/0:0) and LPE(20:5) | 0.7570 | 0.7629 |
| LPE(22:5/0:0) and DPA(22:5n-6) | 0.7611 | 0.7596 |
| LPE(22:5/0:0) and PC(40:8) | 0.7614 | 0.7615 |
| LPE(22:5/0:0) and LPE(18:2/0:0) | 0.7644 | 0.7655 |
| LPE(22:5/0:0) and PC(18:0/18:1) | 0.7566 | 0.7515 |
| DPA(22:5n-6) and LPE(20:5) | 0.7888 | 0.7689 |
| DPA(22:5n-6) and PE(16:1e/20:5) | 0.7875 | 0.7764 |
| DPA(22:5n-6) and PC(40:8) | 0.7879 | 0.7715 |
| DPA(22:5n-6) and PC(18:0/18:2) | 0.7639 | 0.7556 |
| DPA(22:5n-6) and LPE(22:5/0:0) | 0.7617 | 0.7612 |
| DPA(22:5n-6) and LPE(18:2/0:0) | 0.7641 | 0.7654 |
| DPA(22:5n-6) and PC(18:0/18:1) | 0.7574 | 0.7517 |
| LPE(18:2/0:0) and PC(18:0/18:2) | 0.7620 | 0.7499 |
| LPE(18:2/0:0) and PC(18:0/18:1) | 0.7539 | 0.7611 |
| LPE(18:2/0:0) and PE(16:1 e/20:5) | 0.7527 | 0.7607 |
| LPE(18:2/0:0) and PC(40:8) | 0.7542 | 0.7635 |
| LPE(18:2/0:0) and LPE(22:5/0:0) | 0.7556 | 0.7588 |
| LPE(18:2/0:0) and DPA(22:5n-6) | 0.7542 | 0.7679 |
| LPE(18:2/0:0) and LPE(20:5) | 0.7552 | 0.7593 |

**Combinations of three metabolites**

| **Metabolites** | **Average AUC Train** | **Average AUC Test** |
|---|---|---|
| LPE(20:5), PE(16:1e/20:5), DPA(22:5n-6) | 0.7880 | 0.7751 |
| LPE(20:5), PC(40:8), DPA(22:5n-6) | 0.7868 | 0.7716 |
| PE(16:1e/20:5), PC(18:0/18:2), DPA(22:5n-6) | 0.7598 | 0.7714 |
| LPE(20:5), PC(40:8), PC(18:0/18:2) | 0.7585 | 0.7668 |
| PC(40:8), PC(18:0/18:2), DPA(22:5n-6) | 0.7592 | 0.7645 |
| PC(18:0/18:2), LPE(22:5/0:0), DPA(22:5n-6)') | 0.7581 | 0.7592 |
| PC(40:8), PC(18:0/18:2), LPE(18:2/0:0) | 0.7607 | 0.7590 |
| PE(16:1e/20:5), PC(40:8), PC(18:0/18:2) | 0.7579 | 0.7585 |
| PE(16:1e/20:5), PC(18:0/18:2), LPE(22:5/0:0) | 0.7564 | 0.7555 |
| PC(18:0/18:2), LPE(22:5/0:0), LPE(18:2/0:0) | 0.7624 | 0.7554 |
| PC(40:8), PC(18:0/18:2), LPE(22:5/0:0) | 0.7589 | 0.7549 |
| PE(16:1e/20:5), PC(18:0/18:1), PC(18:0/18:2) | 0.7544 | 0.7543 |
| LPE(20:5), PE(16:1e/20:5), PC(18:0/18:2) | 0.7624 | 0.7537 |
| LPE(20:5), PC(18:0/18:2), LPE(18:2/0:0) | 0.7632 | 0.7535 |
| PC(18:0/18:2), DPA(22:5n-6), LPE(18:2/0:0) | 0.7630 | 0.7530 |
| LPE(20:5), PC(18:0/18:2), DPA(22:5n-6) | 0.7632 | 0.7528 |
| PC(18:0/18:1), PC(18:0/18:2), DPA(22:5n-6) | 0.7553 | 0.7510 |
| LPE(20:5), PC(18:0/18:2), LPE(22:5/0:0) | 0.7572 | 0.7509 |
| PC(18:0/18:1), PC(18:0/18:2), LPE(22:5/0:0) | 0.7539 | 0.7507 |
| PE(16:1e/20:5), PC(18:0/18:2), LPE(18:2/0:0) | 0.7615 | 0.7500 |
| LPE(20:5), PC(18:0/18:1), PC(18:0/18:2) | 0.7587 | 0.7473 |
| PC(40:8), LPE(22:5/0:0), LPE(18:2/0:0) | 0.7624 | 0.7456 |
| LPE(22:5/0:0), DPA(22:5n-6), LPE(18:2/0:0) | 0.7656 | 0.7442 |
| PC(40:8), PC(18:0/18:1), PC(18:0/18:2) | 0.7564 | 0.7416 |
| LPE(20:5), LPE(22:5/0:0), LPE(18:2/0:0) | 0.7685 | 0.7396 |
| LPE(20:5), PE(16:1e/20:5), PC(40:8) | 0.7553 | 0.7396 |
| PC(18:0/18:1), PC(18:0/18:2), LPE(18:2/0:0) | 0.7609 | 0.7387 |
| PE(16:1e/20:5), LPE(22:5/0:0), LPE(18:2/0:0) | 0.7649 | 0.7358 |
| LPE(20:5), PC(18:0/18:1), LPE(18:2/0:0) | 0.7357 | 0.7327 |
| PC(18:0/18:1), LPE(22:5/0:0), LPE(18:2/0:0) | 0.7374 | 0.7310 |
| LPE(20:5), PC(18:0/18:1), DPA(22:5n-6) | 0.7264 | 0.7301 |
| LPE(20:5), PC(40:8), PC(18:0/18:1) | 0.7298 | 0.7292 |
| PC(40:8), PC(18:0/18:1), DPA(22:5n-6) | 0.7278 | 0.7287 |
| PC(18:0/18:1), DPA(22:5n-6), LPE(18:2/0:0) | 0.7350 | 0.7287 |
| PE(16:1e/20:5), PC(18:0/18:1), DPA(22:5n-6) | 0.7264 | 0.7278 |
| PE(16:1e/20:5), PC(40:8), PC(18:0/18:1) | 0.7296 | 0.7267 |
| LPE(20:5), PC(40:8), LPE(18:2/0:0) | 0.7259 | 0.7262 |
| PC(40:8), PC(18:0/18:1), LPE(18:2/0:0) | 0.7346 | 0.7260 |
| LPE(20:5), PC(18:0/18:1), LPE(22:5/0:0) | 0.7278 | 0.7254 |
| LPE(20:5), PE(16:1e/20:5), LPE(18:2/0:0) | 0.7289 | 0.7237 |
| PE(16:1e/20:5), PC(18:0/18:1), LPE(22:5/0:0) | 0.7269 | 0.7234 |
| LPE(20:5), PE(16:1e/20:5), PC(18:0/18:1) | 0.7281 | 0.7217 |
| PC(40:8), PC(18:0/18:1), LPE(22:5/0:0) | 0.7282 | 0.7205 |
| PC(18:0/18:1), LPE(22:5/0:0), DPA(22:5n-6) | 0.7269 | 0.7202 |
| PE(16:1e/20:5), PC(18:0/18:1), LPE(18:2/0:0) | 0.7387 | 0.7149 |
| LPE(20:5), DPA(22:5n-6), LPE(18:2/0:0) | 0.7320 | 0.7112 |
| LPE(20:5), LPE(22:5/0:0), DPA(22:5n-6) | 0.7309 | 0.7069 |
| PE(16:1e/20:5), PC(40:8), DPA(22:5n-6) | 0.6973 | 0.7065 |
| PC(40:8), DPA(22:5n-6), LPE(18:2/0:0) | 0.7167 | 0.7011 |
| PC(40:8), LPE(22:5/0:0), DPA(22:5n-6) | 0.7242 | 0.7007 |
| PE(16:1e/20:5), LPE(22:5/0:0), DPA(22:5n-6) | 0.7271 | 0.6992 |
| PE(16:1e/20:5), DPA(22:5n-6), LPE(18:2/0:0) | 0.7176 | 0.6920 |
| PE(16:1e/20:5), PC(40:8), LPE(18:2/0:0) | 0.6943 | 0.6819 |
| PE(16:1e/20:5), PC(40:8), LPE(22:5/0:0) | 0.6836 | 0.6812 |
| LPE(20:5), PC(40:8), LPE(22:5/0:0) | 0.6822 | 0.6776 |
| LPE(20:5), PE(16:1e/20:5), LPE(22:5/0:0) | 0.6833 | 0.6769 |

**Combinations of four metabolites**

| **Metabolites** | **Average AUC Train** | **Average AUC Test** |
|---|---|---|
| LPE(20:5), PE(16:1e/20:5), PC(40:8), DPA(22:5n-6) | 0.7874 | 0.7747 |
| LPE(20:5), PE(16:1e/20:5), PC(18:0/18:2), DPA(22:5n-6) | 0.7599 | 0.7716 |
| LPE(20:5), PC(40:8), PC(18:0/18:2), DPA(22:5n-6) | 0.7588 | 0.7681 |
| LPE(20:5), PC(40:8), PC(18:0/18:2), LPE(18:2/0:0) | 0.7607 | 0.7657 |
| LPE(20:5), PC(18:0/18:2), LPE(22:5/0:0), LPE(18:2/0:0) | 0.7638 | 0.7647 |
| PC(40:8), PC(18:0/18:2), LPE(22:5/0:0), DPA(22:5n-6) | 0.7592 | 0.7638 |
| PC(18:0/18:2), LPE(22:5/0:0), DPA(22:5n-6), LPE(18:2/0:0) | 0.7634 | 0.7627 |
| PE(16:1e/20:5), PC(18:0/18:2), LPE(22:5/0:0), LPE(18:2/0:0) | 0.7613 | 0.7615 |
| PC(40:8), PC(18:0/18:1), PC(18:0/18:2), LPE(18:2/0:0) | 0.7552 | 0.7614 |
| LPE(20:5), PC(18:0/18:1), PC(18:0/18:2), LPE(18:2/0:0) | 0.7586 | 0.7598 |
| LPE(20:5), PE(16:1e/20:5), PC(40:8), PC(18:0/18:2) | 0.7616 | 0.7596 |
| LPE(20:5), PE(16:1e/20:5), PC(18:0/18:2), LPE(22:5/0:0) | 0.7593 | 0.7593 |
| PE(16:1e/20:5), PC(18:0/18:1), PC(18:0/18:2), DPA(22:5n-6) | 0.7548 | 0.7592 |
| LPE(20:5), PC(18:0/18:2), LPE(22:5/0:0), DPA(22:5n-6) | 0.7614 | 0.7591 |
| PC(40:8), PC(18:0/18:2), LPE(22:5/0:0), LPE(18:2/0:0) | 0.7635 | 0.7588 |
| PE(16:1e/20:5), PC(40:8), PC(18:0/18:2), LPE(18:2/0:0) | 0.7618 | 0.7577 |
| PE(16:1e/20:5), PC(18:0/18:1), PC(18:0/18:2), LPE(22:5/0:0) | 0.7540 | 0.7571 |
| LPE(20:5), PC(40:8), PC(18:0/18:2), LPE(22:5/0:0) | 0.7596 | 0.7569 |
| PE(16:1e/20:5), PC(18:0/18:2), LPE(22:5/0:0), DPA(22:5n-6) | 0.7631 | 0.7568 |
| LPE(20:5), PE(16:1e/20:5), PC(18:0/18:2), LPE(18:2/0:0) | 0.7649 | 0.7562 |
| PC(40:8), PC(18:0/18:1), PC(18:0/18:2), DPA(22:5n-6) | 0.7544 | 0.7549 |
| PE(16:1e/20:5), PC(40:8), PC(18:0/18:2), DPA(22:5n-6) | 0.7630 | 0.7542 |
| LPE(20:5), PC(18:0/18:2), DPA(22:5n-6), LPE(18:2/0:0) | 0.7654 | 0.7538 |
| PE(16:1e/20:5), PC(40:8), PC(18:0/18:2), LPE(22:5/0:0) | 0.7606 | 0.7537 |
| PC(18:0/18:1), PC(18:0/18:2), LPE(22:5/0:0), DPA(22:5n-6) | 0.7549 | 0.7530 |
| LPE(20:5), PC(18:0/18:1), PC(18:0/18:2), DPA(22:5n-6) | 0.7563 | 0.7527 |
| LPE(20:5), PE(16:1e/20:5), LPE(22:5/0:0), LPE(18:2/0:0) | 0.7641 | 0.7519 |
| PE(16:1e/20:5), PC(18:0/18:2), DPA(22:5n-6), LPE(18:2/0:0) | 0.7636 | 0.7513 |
| LPE(20:5), PC(40:8), PC(18:0/18:1), PC(18:0/18:2) | 0.7564 | 0.7500 |
| PE(16:1e/20:5), PC(40:8), LPE(22:5/0:0), LPE(18:2/0:0) | 0.7611 | 0.7497 |
| PC(40:8), PC(18:0/18:2), DPA(22:5n-6), LPE(18:2/0:0) | 0.7662 | 0.7497 |
| PC(40:8), PC(18:0/18:1), PC(18:0/18:2), LPE(22:5/0:0) | 0.7554 | 0.7481 |
| PE(16:1e/20:5), PC(40:8), PC(18:0/18:1), PC(18:0/18:2) | 0.7554 | 0.7467 |
| PC(40:8), LPE(22:5/0:0), DPA(22:5n-6), LPE(18:2/0:0) | 0.7664 | 0.7456 |
| LPE(20:5), PC(18:0/18:1), PC(18:0/18:2), LPE(22:5/0:0) | 0.7572 | 0.7454 |
| PC(18:0/18:1), PC(18:0/18:2), DPA(22:5n-6), LPE(18:2/0:0) | 0.7624 | 0.7448 |
| LPE(20:5), PC(40:8), LPE(22:5/0:0), LPE(18:2/0:0) | 0.7667 | 0.7447 |
| LPE(20:5), LPE(22:5/0:0), DPA(22:5n-6), LPE(18:2/0:0) | 0.7703 | 0.7442 |
| PE(16:1e/20:5), PC(18:0/18:1), PC(18:0/18:2), LPE(18:2/0:0) | 0.7570 | 0.7439 |
| LPE(20:5), PE(16:1e/20:5), PC(18:0/18:1), PC(18:0/18:2) | 0.7599 | 0.7419 |
| PE(16:1e/20:5), PC(40:8), PC(18:0/18:1), LPE(18:2/0:0) | 0.7318 | 0.7417 |
| PC(18:0/18:1), PC(18:0/18:2), LPE(22:5/0:0), LPE(18:2/0:0) | 0.7600 | 0.7408 |
| LPE(20:5), PE(16:1e/20:5), PC(18:0/18:1), LPE(18:2/0:0) | 0.7324 | 0.7399 |
| PC(40:8), PC(18:0/18:1), DPA(22:5n-6), LPE(18:2/0:0) | 0.7327 | 0.7359 |
| LPE(20:5), PC(40:8), PC(18:0/18:1), LPE(18:2/0:0) | 0.7325 | 0.7357 |
| PE(16:1e/20:5), LPE(22:5/0:0), DPA(22:5n-6), LPE(18:2/0:0) | 0.7692 | 0.7349 |
| PE(16:1e/20:5), PC(18:0/18:1), LPE(22:5/0:0), LPE(18:2/0:0) | 0.7364 | 0.7342 |
| LPE(20:5), PC(18:0/18:1), DPA(22:5n-6), LPE(18:2/0:0) | 0.7350 | 0.7321 |
| PC(40:8), PC(18:0/18:1), LPE(22:5/0:0), LPE(18:2/0:0) | 0.7372 | 0.7313 |
| PE(16:1e/20:5), PC(40:8), PC(18:0/18:1), LPE(22:5/0:0) | 0.7254 | 0.7312 |
| PC(18:0/18:1), LPE(22:5/0:0), DPA(22:5n-6), LPE(18:2/0:0) | 0.7354 | 0.7310 |
| LPE(20:5), PE(16:1E/20:5), PC(40:8), PC(18:0/18:1) | 0.7270 | 0.7302 |
| LPE(20:5), PC(40:8), PC(18:0/18:1), LPE(22:5/0:0) | 0.7255 | 0.7295 |
| PE(16:1e/20:5), PC(40:8), PC(18:0/18:1), DPA(22:5n-6) | 0.7276 | 0.7287 |
| PE(16:1e/20:5), PC(18:0/18:1), DPA(22:5n-6), LPE(18:2/0:0) | 0.7335 | 0.7277 |
| LPE(20:5), PE(16:1e/20:5), PC(18:0/18:1), DPA(22:5n-6) | 0.7290 | 0.7273 |
| LPE(20:5), PC(18:0/18:1), LPE(22:5/0:0), DPA(22:5n-6) | 0.7255 | 0.7263 |
| LPE(20:5), PC(18:0/18:1), LPE(22:5/0:0), LPE(18:2/0:0) | 0.7399 | 0.7256 |
| PE(16:1e/20:5), PC(18:0/18:1), LPE(22:5/0:0), DPA(22:5n-6) | 0.7269 | 0.7228 |
| LPE(20:5), PC(40:8), PC(18:0/18:1), DPA(22:5n-6) | 0.7306 | 0.7208 |
| PC(40:8), PC(18:0/18:1), LPE(22:5/0:0), DPA(22:5n-6) | 0.7283 | 0.7198 |
| LPE(20:5), PE(16:1e/20:5), PC(18:0/18:1), LPE(22:5/0:0) | 0.7285 | 0.7185 |
| LPE(20:5), PE(16:1e/20:5), PC(40:8), LPE(18:2/0:0) | 0.7270 | 0.7173 |
| LPE(20:5), PC(40:8), DPA(22:5n-6), LPE(18:2/0:0) | 0.7347 | 0.7111 |
| PE(16:1e/20:5), PC(40:8), LPE(22:5/0:0), DPA(22:5n-6) | 0.7241 | 0.7106 |
| LPE(20:5), PE(16:1e/20:5), DPA(22:5n-6), LPE(18:2/0:0) | 0.7354 | 0.7057 |
| LPE(20:5), PE(16:1e/20:5), LPE(22:5/0:0), DPA(22:5n-6) | 0.7324 | 0.7012 |
| LPE(20:5), PC(40:8), LPE(22:5/0:0), DPA(22:5n-6) | 0.7295 | 0.6931 |
| PE(16:1e/20:5), PC(40:8), DPA(22:5n-6), LPE(18:2/0:0) | 0.7190 | 0.6894 |
| LPE(20:5), PE(16:1e/20:5), PC(40:8), LPE(22:5/0:0) | 0.6805 | 0.6857 |

**Combinations of five metabolites**

| **Metabolites** | **Average AUC Train** | **Average AUC Test** |
|---|---|---|
| PC(40:8), PC(18:0/18:2), LPE(22:5/0:0), DPA(22:5n-6), LPE(18:2/0:0) | 0.7595 | 0.7758 |
| LPE(20:5), PE(16:1e/20:5), PC(18:0/18:1), PC(18:0/18:2), LPE(18:2/0:0) | 0.7557 | 0.7647 |
| LPE(20:5), PC(40:8), PC(18:0/18:2), LPE(22:5/0:0), LPE(18:2/0:0) | 0.7627 | 0.7646 |
| LPE(20:5), PC(40:8), PC(18:0/18:2), LPE(22:5/0:0), DPA(22:5n-6) | 0.7581 | 0.7628 |
| PC(40:8), PC(18:0/18:1), PC(18:0/18:2), DPA(22:5n-6), LPE(18:2/0:0) | 0.7554 | 0.7624 |
| PE(16:1e/20:5), PC(40:8), PC(18:0/18:2), LPE(22:5/0:0), LPE(18:2/0:0) | 0.7634 | 0.7615 |
| PE(16:1e/20:5), PC(18:0/18:2), LPE(22:5/0:0), DPA(22:5n-6), LPE(18:2/0:0) | 0.7614 | 0.7614 |
| PE(16:1e/20:5), PC(40:8), PC(18:0/18:2), DPA(22:5n-6), LPE(18:2/0:0) | 0.7643 | 0.7606 |
| LPE(20:5), PE(16:1e/20:5), PC(40:8), PC(18:0/18:2), DPA(22:5n-6) | 0.7598 | 0.7603 |
| LPE(20:5), PC(40:8), PC(18:0/18:2), DPA(22:5n-6), LPE(18:2/0:0) | 0.7631 | 0.7601 |
| PE(16:1e/20:5), PC(40:8), PC(18:0/18:2), LPE(22:5/0:0), DPA(22:5n-6) | 0.7604 | 0.7592 |
| LPE(20:5), PE(16:1e/20:5), PC(18:0/18:2), LPE(22:5/0:0), LPE(18:2/0:0) | 0.7621 | 0.7591 |
| LPE(20:5), PC(18:0/18:1), PC(18:0/18:2), LPE(22:5/0:0), DPA(22:5n-6) | 0.7554 | 0.7590 |
| PE(16:1e/20:5), PC(40:8), PC(18:0/18:1), PC(18:0/18:2), LPE(18:2/0:0) | 0.7573 | 0.7583 |
| LPE(20:5), PC(18:0/18:2), LPE(22:5/0:0), DPA(22:5n-6), LPE(18:2/0:0) | 0.7662 | 0.7574 |
| PC(18:0/18:1), PC(18:0/18:2), LPE(22:5/0:0), DPA(22:5n-6), LPE(18:2/0:0) | 0.7559 | 0.7574 |
| LPE(20:5), PE(16:1e/20:5), PC(18:0/18:2), LPE(22:5/0:0), DPA(22:5n-6) | 0.7584 | 0.7565 |
| LPE(20:5), PC(18:0/18:1), PC(18:0/18:2), LPE(22:5/0:0), LPE(18:2/0:0) | 0.7578 | 0.7554 |
| LPE(20:5), PE(16:1e/20:5), PC(40:8), PC(18:0/18:1), PC(18:0/18:2) | 0.7554 | 0.7553 |
| LPE(20:5), PC(40:8), PC(18:0/18:1), PC(18:0/18:2), DPA(22:5n-6) | 0.7572 | 0.7540 |
| LPE(20:5), PE(16:1e/20:5), PC(40:8), PC(18:0/18:2), LPE(18:2/0:0) | 0.7651 | 0.7537 |
| PE(16:1e/20:5), PC(40:8), PC(18:0/18:1), PC(18:0/18:2), DPA(22:5n-6) | 0.7580 | 0.7529 |
| PC(40:8), PC(18:0/18:1), PC(18:0/18:2), LPE(22:5/0:0), LPE(18:2/0:0) | 0.7590 | 0.7522 |
| LPE(20:5), PE(16:1e/20:5), PC(40:8), LPE(22:5/0:0), LPE(18:2/0:0) | 0.7626 | 0.7521 |
| LPE(20:5), PE(16:1e/20:5), LPE(22:5/0:0), DPA(22:5n-6), LPE(18:2/0:0) | 0.7695 | 0.7518 |
| LPE(20:5), PC(40:8), PC(18:0/18:1), PC(18:0/18:2), LPE(18:2/0:0) | 0.7580 | 0.7515 |
| PE(16:1e/20:5), PC(18:0/18:1), PC(18:0/18:2), LPE(22:5/0:0), DPA(22:5n-6) | 0.7570 | 0.7507 |
| LPE(20:5), PE(16:1e/20:5), PC(18:0/18:1), PC(18:0/18:2), DPA(22:5n-6) | 0.7590 | 0.7506 |
| LPE(20:5), PE(16:1e/20:5), PC(40:8), PC(18:0/18:2), LPE(22:5/0:0) | 0.7624 | 0.7504 |
| PC(40:8), PC(18:0/18:1), PC(18:0/18:2), LPE(22:5/0:0), DPA(22:5n-6) | 0.7578 | 0.7504 |
| PE(16:1e/20:5), PC(40:8), PC(18:0/18:1), PC(18:0/18:2), LPE(22:5/0:0) | 0.7577 | 0.7489 |
| PE(16:1e/20:5), PC(18:0/18:1), PC(18:0/18:2), DPA(22:5n-6), LPE(18:2/0:0) | 0.7608 | 0.7476 |
| PE(16:1e/20:5), PC(18:0/18:1), PC(18:0/18:2), LPE(22:5/0:0), LPE(18:2/0:0) | 0.7607 | 0.7476 |
| LPE(20:5), PE(16:1e/20:5), PC(18:0/18:2), DPA(22:5n-6), LPE(18:2/0:0) | 0.7667 | 0.7473 |
| PE(16:1e/20:5), PC(40:8), LPE(22:5/0:0), DPA(22:5n-6), LPE(18:2/0:0) | 0.7649 | 0.7446 |
| LPE(20:5), PC(18:0/18:1), PC(18:0/18:2), DPA(22:5n-6), LPE(18:2/0:0) | 0.7600 | 0.7437 |
| LPE(20:5), PC(40:8), PC(18:0/18:1), PC(18:0/18:2), LPE(22:5/0:0) | 0.7608 | 0.7433 |
| LPE(20:5), PE(16:1e/20:5), PC(18:0/18:1), PC(18:0/18:2), LPE(22:5/0:0) | 0.7597 | 0.7404 |
| LPE(20:5), PE(16:1e/20:5), PC(18:0/18:1), LPE(22:5/0:0), LPE(18:2/0:0) | 0.7347 | 0.7391 |
| LPE(20:5), PC(40:8), LPE(22:5/0:0), DPA(22:5n-6), LPE(18:2/0:0) | 0.7725 | 0.7368 |
| LPE(20:5), PE(16:1e/20:5), PC(18:0/18:1), DPA(22:5n-6), LPE(18:2/0:0) | 0.7352 | 0.7364 |
| PE(16:1e/20:5), PC(18:0/18:1), LPE(22:5/0:0), DPA(22:5n-6), LPE(18:2/0:0) | 0.7354 | 0.7328 |
| PE(16:1e/20:5), PC(40:8), PC(18:0/18:1), DPA(22:5n-6), LPE(18:2/0:0) | 0.7341 | 0.7327 |
| PC(40:8), PC(18:0/18:1), LPE(22:5/0:0), DPA(22:5n-6), LPE(18:2/0:0) | 0.7383 | 0.7294 |
| LPE(20:5), PC(40:8), PC(18:0/18:1), LPE(22:5/0:0), LPE(18:2/0:0) | 0.7379 | 0.7292 |
| LPE(20:5), PC(40:8), PC(18:0/18:1), DPA(22:5n-6), LPE(18:2/0:0) | 0.7350 | 0.7286 |
| LPE(20:5), PE(16:1e/20:5), PC(40:8), PC(18:0/18:1), LPE(18:2/0:0) | 0.7351 | 0.7273 |
| LPE(20:5), PC(40:8), PC(18:0/18:1), LPE(22:5/0:0), DPA(22:5n-6) | 0.7278 | 0.7269 |
| LPE(20:5), PE(16:1e/20:5), PC(40:8), PC(18:0/18:1), DPA(22:5n-6) | 0.7297 | 0.7265 |
| LPE(20:5), PE(16:1e/20:5), PC(40:8), PC(18:0/18:1), LPE(22:5/0:0) | 0.7276 | 0.7258 |
| LPE(20:5), PC(18:0/18:1), LPE(22:5/0:0), DPA(22:5n-6), LPE(18:2/0:0) | 0.7385 | 0.7240 |
| LPE(20:5), PE(16:1e/20:5), PC(18:0/18:1), LPE(22:5/0:0), DPA(22:5n-6) | 0.7279 | 0.7238 |
| PE(16:1e/20:5), PC(40:8), PC(18:0/18:1), LPE(22:5/0:0), LPE(18:2/0:0) | 0.7410 | 0.7224 |
| LPE(20:5), PE(16:1e/20:5), PC(40:8), DPA(22:5n-6), LPE(18:2/0:0) | 0.7348 | 0.7203 |
| PE(16:1e/20:5), PC(40:8), PC(18:0/18:1), LPE(22:5/0:0), DPA(22:5n-6) | 0.7315 | 0.7139 |
| LPE(20:5), PE(16:1e/20:5), PC(40:8), LPE(22:5/0:0), DPA(22:5n-6) | 0.7309 | 0.7096 |

### Example 4: Discussion

In this study, the inventors have performed a comparative analysis of the lipidomic patterns of implantative and non-implantative endometrial fluid. The advantage of using a metabolomics approach in the study of endometrial fluid samples which has been aspirated at the very same time as embryo transfer is especially interesting, due to the strength link between the metabolites and the phenotype. The metabolomics profiling aims to provide a close real-time assessment of a specific metabolic state.

Only eight metabolites were significantly altered in women with a non-implantative endometrial fluid, but remarkably, all of them were glycerophospholipids except an omega-6 PUFA. The levels of the lysophosphatidylethanolamines LPE(22:5/0:0), LPE(18:2/0:0) and LPE(20:5), the ether-linked phosphatidylethanolamine PE(16:1e/20:5) and the diacylphosphatidylcholines PC(18:0/18:1) and PC(18:0/18:2) decreased in the endometrial fluid patients with a failure in the implantation outcome. In contrast, the docosapentaenoic acid (22:5n-6) and the diacylphosphatidylcholine PC(40:8) decreased.

Even though few significant differences were obtained when the lipid species were evaluated individually, a predictive lipidomic signature linked to the implantative status of the endometrial fluid has been found. Then, the levels of the highlighted lipids were associated with the presence of alterations in the endometrial fluid linked to implantation failures, and although the causal role of these metabolites cannot be determined, they probably reflect alterations in key pathways. Indeed, women with a non-implantative EFA are accurately classified with a SVM algorithm including the eight lipid metabolites selected in the univariate analysis. This classification model yielded an AUC of 0.893±0.07, 85.7% sensitivity, 80.0% specificity and 82.8% accuracy.

## Claims

1. A method for predicting the outcome of, or assessing the chances of success of, in vitro fertilisation (IVF) in a subject, wherein the method comprises determining, in an endometrial fluid sample from the subject, the expression level of at least one lipid marker selected from the group consisting of
• Lysoglycerophosphoethanolamine (20:5);
• 1-ether, 2-acylglycerophosphoethanolamine (16:1e/20:5);
• Diacylglycerophosphocholine (40:8);
• Diacylglycerophosphocholine (18:0/18:1);
• Diacylglycerophosphocholine (18:0/18:2);
• Lysoglycerophosphoethanolamine (22:5/0:0);
• Docosapentaenoic acid (22:5n-6); and/ or
• Lysoglycerophosphoethanolamine (18:2/0:0),
wherein
• a decrease in the expression level of Diacylglycerophosphocholine (40:8) and Docosapentaenoic acid (22:5n-6) with respect to a reference value, and
• an increase in the expression level of Lysoglycerophosphoethanolamine (20:5); 1-ether, 2-acylglycerophosphoethanolamine (16:1e/20:5); Diacylglycerophosphocholine (18:0/18:1); Diacylglycerophosphocholine (18:0/18:2); Lysoglycerophosphoethanolamine (22:5/0:0); and Lysoglycerophosphoethanolamine (18:2/0:0) with respect to a reference value
is indicative of an increased chance of a positive outcome of IVF for the subject.

2. A method for selecting a subject for implantation with at least one embryo during IVF, wherein the method comprises determining, in an endometrial fluid sample from the subject, the expression level of at least one lipid marker selected from the group consisting of
• Lysoglycerophosphoethanolamine (20:5);
• 1-ether, 2-acylglycerophosphoethanolamine (16:1e/20:5);
• Diacylglycerophosphocholine (40:8);
• Diacylglycerophosphocholine (18:0/18:1);
• Diacylglycerophosphocholine (18:0/18:2);
• Lysoglycerophosphoethanolamine (22:5/0:0);
• Docosapentaenoic acid (22:5n-6); and/ or
• Lysoglycerophosphoethanolamine (18:2/0:0),
wherein
• a decrease in the expression level of Diacylglycerophosphocholine (40:8) and Docosapentaenoic acid (22:5n-6) with respect to a reference value, and
• an increase in the expression level of Lysoglycerophosphoethanolamine (20:5); 1-ether, 2-acylglycerophosphoethanolamine (16:1e/20:5); Diacylglycerophosphocholine (18:0/18:1); Diacylglycerophosphocholine (18:0/18:2); Lysoglycerophosphoethanolamine (22:5/0:0); and Lysoglycerophosphoethanolamine (18:2/0:0) with respect to a reference value
is indicative that the subject is selected for implantation with one embryo,
and wherein
• an increase in the expression level of Diacylglycerophosphocholine (40:8) and Docosapentaenoic acid (22:5n-6) with respect to a reference value, and
• a decrease in the expression level of Lysoglycerophosphoethanolamine (20:5); 1-ether, 2-acylglycerophosphoethanolamine (16:1e/20:5); Diacylglycerophosphocholine (18:0/18:1); Diacylglycerophosphocholine (18:0/18:2); Lysoglycerophosphoethanolamine (22:5/0:0); and Lysoglycerophosphoethanolamine (18:2/0:0) with respect to a reference value
is indicative that the subject is selected for implantation with two or more embryos, or that the embryos are frozen and IVF is postponed.

3. A method for selecting an IVF treatment for a subject, wherein the method comprises determining, in an endometrial fluid sample from the subject, the expression level of at least one lipid marker selected from the group consisting of
• Lysoglycerophosphoethanolamine (20:5);
• 1-ether, 2-acylglycerophosphoethanolamine (16:1e/20:5);
• Diacylglycerophosphocholine (40:8);
• Diacylglycerophosphocholine (18:0/18:1);
• Diacylglycerophosphocholine (18:0/18:2);
• Lysoglycerophosphoethanolamine (22:5/0:0);
• Docosapentaenoic acid (22:5n-6); and/ or
• Lysoglycerophosphoethanolamine (18:2/0:0),
wherein
• a decrease in the expression level of Diacylglycerophosphocholine (40:8) and Docosapentaenoic acid (22:5n-6) with respect to a reference value, and
• an increase in the expression level of Lysoglycerophosphoethanolamine (20:5); 1-ether, 2-acylglycerophosphoethanolamine (16:1e/20:5); Diacylglycerophosphocholine (18:0/18:1); Diacylglycerophosphocholine (18:0/18:2); Lysoglycerophosphoethanolamine (22:5/0:0); and Lysoglycerophosphoethanolamine (18:2/0:0) with respect to a reference value
is indicative that an IVF treatment comprising the implantation of one embryo is selected for the subject,
and wherein
• an increase in the expression level of Diacylglycerophosphocholine (40:8) and Docosapentaenoic acid (22:5n-6) with respect to a reference value, and
• a decrease in the expression level of Lysoglycerophosphoethanolamine (20:5); 1-ether, 2-acylglycerophosphoethanolamine (16:1e/20:5); Diacylglycerophosphocholine (18:0/18:1); Diacylglycerophosphocholine (18:0/18:2); Lysoglycerophosphoethanolamine (22:5/0:0); and Lysoglycerophosphoethanolamine (18:2/0:0) with respect to a reference value
is indicative that an IVF treatment comprising the implantation of two or more embryos is selected for the subject, or that the embryos are frozen and IVF is postponed.

4. The method according to any one of claims 1 to 3, wherein the method comprises determining markers
• 1-ether, 2-acylglycerophosphoethanolamine (16:1e/20:5); and
• Diacylglycerophosphocholine (40:8).

5. The method according to any one of claims 1 to 3, wherein the method comprises determining all of lipid markers
• Lysoglycerophosphoethanolamine (20:5);
• 1-ether, 2-acylglycerophosphoethanolamine (16:1e/20:5);
• Diacylglycerophosphocholine (40:8);
• Diacylglycerophosphocholine (18:0/18:1);
• Diacylglycerophosphocholine (18:0/18:2);
• Lysoglycerophosphoethanolamine (22:5/0:0);
• Docosapentaenoic acid (22:5n-6); and
• Lysoglycerophosphoethanolamine (18:2/0:0).

6. The method according to any one of claims 1 to 5, wherein the expression level of the at least one lipid marker is determined by UHPLC-ToF-MS.

7. The method according to any one of claims 1 to 6, wherein the subject has undergone a standard IVF cycle.

8. The method according to any one of claims 1 to 7, wherein the endometrial fluid sample is collected as an endometrial fluid aspirate.

9. The method according to any one of claims 1 to 8, wherein the subject is a woman.

10. Use of reagents specific for the determination of the expression levels of
• Lysoglycerophosphoethanolamine (20:5);
• 1-ether, 2-acylglycerophosphoethanolamine (16:1e/20:5);
• Diacylglycerophosphocholine (40:8);
• Diacylglycerophosphocholine (18:0/18:1);
• Diacylglycerophosphocholine (18:0/18:2);
• Lysoglycerophosphoethanolamine (22:5/0:0);
• Docosapentaenoic acid (22:5n-6); and/ or
• Lysoglycerophosphoethanolamine (18:2/0:0),
for predicting the outcome of, or assessing the chances of success of, IVF in a subject.

11. An endometrial fluid wash solution for use in the treatment of infertility in a subject, wherein the wash solution is suitable for
• decreasing the expression level of Diacylglycerophosphocholine (40:8) and Docosapentaenoic acid (22:5n-6) with respect to a reference value, and
• increasing the expression level of Lysoglycerophosphoethanolamine (20:5); 1-ether, 2-acylglycerophosphoethanolamine (16:1e/20:5); Diacylglycerophosphocholine (18:0/18:1); Diacylglycerophosphocholine (18:0/18:2); Lysoglycerophosphoethanolamine (22:5/0:0); and Lysoglycerophosphoethanolamine (18:2/0:0) with respect to a reference value.

12. The endometrial fluid wash solution for use according to claim 11, wherein the solution is a physiological saline solution.

13. The endometrial fluid wash solution for use according to any one of claims 11 or 12, wherein the solution comprises an effective amount of
• Lysoglycerophosphoethanolamine (20:5);
• 1-ether, 2-acylglycerophosphoethanolamine (16:1e/20:5);
• Diacylglycerophosphocholine (40:8);
• Diacylglycerophosphocholine (18:0/18:1);
• Diacylglycerophosphocholine (18:0/18:2);
• Lysoglycerophosphoethanolamine (22:5/0:0);
• Docosapentaenoic acid (22:5n-6); and/ or
• Lysoglycerophosphoethanolamine (18:2/0:0).

14. An endometrial fluid wash solution comprising an effective amount of
• Lysoglycerophosphoethanolamine (20:5);
• 1-ether, 2-acylglycerophosphoethanolamine (16:1e/20:5);
• Diacylglycerophosphocholine (40:8);
• Diacylglycerophosphocholine (18:0/18:1);
• Diacylglycerophosphocholine (18:0/18:2);
• Lysoglycerophosphoethanolamine (22:5/0:0);
• Docosapentaenoic acid (22:5n-6); and/ or
• Lysoglycerophosphoethanolamine (18:2/0:0).
